# EUROPEAN PATENT SPECIFICATION

(11) **EP 1 673 473 B1**
(45) Date of publication and mention of the grant of the patent: **06.07.2011**
(21) Application number: 04765831.5
(22) Date of filing: 05.10.2004
(51) Int. Cl.: C12Q 1/68

(54) **USE OF GENETIC POLYMORPHISMS THAT ASSOCIATE WITH EFFICACY OF TREATMENT OF INFLAMMATORY DISEASE**
VERWENDUNG VON MIT DER WIRKSAMKEIT DER BEHANDLUNG EINER ENTZÜNDUNGSKRANKHEIT ASSOZIIERTEN GENETISCHEN POLYMORPHISMEN
UTILISATION DE POLYMORPHISMES GENETIQUES COMPATIBLES AVEC L'EFFICACITE DE TRAITEMENT DES MALADIES INFLAMMATOIRES

(30) Priority: 06.10.2003 US 508971 P
(43) Date of publication of application: 28.06.2006
(73) Proprietor: Novartis AG, 4056 Basel (CH); Novartis Pharma GmbH, 1230 Wien (AT)
(72) Inventor: IDE, Susan, Hollis, NH 03049 (US); LAVEDAN, Christian, Nicolas, Potomac, MD 20854 (US); McCULLOUGH, Karen, Olney, MD 20832 (US)
(74) Representative: de Weerd, Petrus G.W.
(86) International application number: PCT/EP2004/011124
(87) International publication number: WO 2005/040416

(56) References cited:
- EP-A- 0 288 088
- WO-A-02/089796
- US-B1- 6 235 723
- HIGUCHI T ET AL: "POLYMORPHISM OF THE 5'-FLANKING REGION OF THE HUMAN TUMOR NECROSIS FACTOR (TNF)-ALPHA GENE IN JAPANESE" TISSUE ANTIGENS, MUNKSGAARD, COPENHAGEN, DK, vol. 51, no. 6, 1998, pages 605-612, XP008020598 ISSN: 0001-2815
- SMITH ET AL: "Contrasting genetic influence of CCR2 and CCR5 variants on HIV -1 infection and disease progression" SCIENCE, AMERICAN ASSOCIATION FOR THE ADVANCEMENT OF SCIENCE,, US, vol. 277, 15 August 1997 (1997-08-15), pages 959-965, XP002099015 ISSN: 0036-8075
- HOLWEG C T J ET AL: "Clinical impact of cytokine gene polymorphisms in heart and lung transplantation" JOURNAL OF HEART AND LUNG TRANSPLANTATION, MOSBY-YEAR BOOK, INC., ST LOUIS, MO, US, vol. 23, no. 9, September 2004 (2004-09), pages 1017-1026, XP004595231 ISSN: 1053-2498
- ABRAHAM L J ET AL: "HAPLOTYPIC POLYMORPHISMS OF THE TNFB GENE" IMMUNOGENETICS, vol. 33, no. 1, 1991, pages 50-53, XP009041878 ISSN: 0093-7711
- LEE BENHUR ET AL: "Influence of the CCR2-V64I polymorphism on human immunodeficiency virus type 1 coreceptor activity and on chemokine receptor function of CCR2b, CCR3, CCR5, and CXCR4" JOURNAL OF VIROLOGY, vol. 72, no. 9, September 1998 (1998-09), pages 7450-7458, XP002312893 ISSN: 0022-538X

## Description

### FIELD OF THE INVENTION

This disclosure relates generally to the analytical testing of tissue samples *in vitro,* and more particularly to the analysis of genetic polymorphisms as biomarkers for the efficacy of treatments of inflammatory disease.

### DESCRIPTION OF THE RELATED ART

Atopic dermatitis (AD), also known as eczema, is an inflammatory skin disease typified by itchy inflamed skin that presents during infancy and childhood. The disease is one of the most common dermatological disorders in Western countries, affecting as many as 20% of children under the age of five. Cookson WO et al., Nature Genetics 27: 372-373 (2001).

Pimecrolimus (Elidel®) is an ascomycin macrolactam derivative that was specifically developed for the treatment of inflammatory skin diseases. Tacrolimus (FK506; Protopic® or Prograf®) is a member of the same class of compounds and is also used to treat inflammatory skin diseases. Both pimecrolimus and tacrolimus interfere with the inflammatory process by binding with high affinity to macrophilin-12 and inhibiting calcineurin, which blocks transcription of Th1 - and Th2-type cytokines in T lymphocytes. While the two compounds are structurally similar and have comparable mechanisms of action, pimecrolimus has a higher affinity for skin than does tacrolimus.

It has long been recognized that there is a genetic basis to atopic diseases. Several linkage analyses have been done to define susceptibility regions that contribute to atopic dermatitis, as reviewed in Maclean JA & Eidelman FJ, Arch Dermatol 137: 1474-1476 (2001). At least five chromosomal regions (3q21, 5q31-33, 11q13, 13q12-14 and 14q11.2) are thought to contain atopic dermatitis susceptibility loci. Current thinking is that atopic mechanisms are primarily responsible for the pathogenesis of the atopic dermatitis. However, other genes involved with dermal inflammation may also contribute to the condition. Cookson WO et al., Nature Genetics 27: 372-373 (2001).

The response rate among patients taking topical macrolactams (pimecrolimus or tacrolimus) is currently less than 50%. It is not known why some patients do not respond to these medications. Thus, there remains a need in the art for the improving the efficacy of topical macrolactams, such as by targeting appropriate formulations of the medications to those inflammatory disease patients who are genetically disposed to respond and benefit from the treatment.

### SUMMARY OF THE INVENTION

The disclosure provides a method for treating atopic dermatitis, based upon a determination of the patient's *TNF* alleles. In one embodiment, patients are tested for the presence of a (-1031) *TNF* polymorphism to determine what therapy is appropriate. Patients having the TT alleles (SEQ ID NO:1) at that locus are treated with pimecrolimus cream or ointment, while patients having the CC alleles (SEQ ID NO:2) or the CT alleles (SEQ ID NOS:1 and 2) are treated with a higher dose of pimecrolimus cream, with pimecrolimus cream plus another anti-inflammatory drug, or with an anti-inflammatory drug without pimecrolimus cream.

The disclosure also provides a general method for treating psoriasis, asthma, inflammatory bowel disease, rheumatoid arthritis or any disorder for which pimecrolimus (oral, topical or other) is indicated, based upon a determination of the patient's *TNF* alleles. In one embodiment, patients are tested for the presence of a (-1031) *TNF* polymorphism to determine what therapy is appropriate. Patients with TT alleles at that locus are treated with pimecrolimus, while patients with CC alleles and CT alleles are given a higher dose of pimecrolimus, pimecrolimus plus another anti-inflammatory drug or an anti-inflammatory drug without pimecrolimus.

The disclosure further provides a method for treating psoriasis, asthma, inflammatory bowel disease, rheumatoid arthritis or any disorder for which pimecrolimus (oral, topical or other) is indicated, based upon a determination of the patient's *LTA* alleles. In one embodiment, patients are tested for the presence of an ASN60THR *LTA* locus polymorphism to determine what therapy is appropriate. Patients with AA alleles (SEQ ID NO:3) or AC alleles (SEQ ID NOS:3 and 4) at that locus are treated with pimecrolimus, while patients with CC alleles (SEQ ID NO:4) are given a higher dose of pimecrolimus, pimecrolimus plus another anti-inflammatory drug or an anti-inflammatory drug without pimecrolimus.

The disclosure also provides a method for treating psoriasis, asthma, inflammatory bowel disease, rheumatoid arthritis or any disorder for which tacrolimus (oral, topical or other) is indicated, based upon a determination of the patient's *CCR2* alleles. In one embodiment, patients are tested for the presence of a VAL64ILE *CCR2* locus polymorphism to determine what therapy is appropriate. Patients with GG alleles (SEQ ID NO:5) at that locus are treated with tacrolimus, while patients with AG alleles (SEQ ID NOS:5 and 6) are given a higher dose of tacrolimus, tacrolimus plus another anti-inflammatory drug or an anti-inflammatory drug without tacrolimus.

The disclosure provides a method for treating atopic dermatitis or any disorder for which pimecrolimus (oral, topical or other) is indicated, where the level of TNF-α protein or mRNA present in the patient is measured prior to determining what therapy is appropriate. In one embodiment, when TNF-α protein or mRNA levels in the bodily fluids or non-inflamed tissue samples of the patient are low or normal, then the patient is treated with pimecrolimus. In another embodiment, when TNF-α protein or mRNA levels are elevated, then the patient is treated with pimecrolimus in combination with another anti-inflammatory drug or with an alternative therapy.

The disclosure further provides a method for determining a treatment strategy for a condition in a subject, wherein the condition is selected from the group consisting of atopic dermatitis, psoriasis, asthma, inflammatory bowel disease, rheumatoid arthritis or other condition for which pimecrolimus is indicated, said method comprises analyzing the level of TNF-α mRNA or TNF-α protein in a sample obtained from the subject,; and wherein the treatment strategy comprises the selection of a pimecrolimus formulation as treatment when the level of TNF-α mRNA or TNF-α protein in the sample is low or normal; or the selection of either (A) a pimecrolimus formulation in combination with another immunosuppressant or (B) another immunosuppressant as treatment when the level of TNF-α mRNA or TNF-α protein in the sample is elevated. Preferably, said sample is taken from non-inflamed tissue.

The disclosure provides a method for choosing subjects for inclusion in a clinical trial of inflammatory skin disease or any other disorder for which pimecrolimus is indicated, where genetic polymorphisms at the (-1031) TNF locus, the ASN60THR *LTA* locus or the VAL64ILE *CCR2* locus of the candidate subjects are interrogated prior to inclusion in the clinical trial.

The disclosure provides a kit for use in determining treatment strategy for a patient with inflammatory skin disease or any other disorder for which pimecrolimus is indicated. In one embodiment, the kit contains reagents for determining the levels of TNF-α protein or mRNA in a sample obtained from a subject to be treated. In another embodiment, the kit contains reagents for determining the genotype of the subject in the *TNF, LTA* or *CCR2* genes. The kit also contains a written product describing the use of the biomarker in determining a treatment strategy for the condition.

The disclosure further provides a method for determining a treatment strategy for a condition in a subject, wherein the condition is selected from the group consisting of atopic dermatitis, psoriasis, asthma, inflammatory bowel disease, rheumatoid arthritis or other condition for which pimecrolimus or tacrolimus is indicated, said method comprises analyzing the genotype of a subject at a genetic locus from the TNF gene cluster indicative of efficacy of a selected macrolactam formulation in treating the condition. Preferably, the genotype is determined by analyzing a sample obtained from said subject. In one embodiment, subjects / patients are tested for the presence of a (-1031) *TNF* polymorphism to determine the appropriate treatment strategy. For patients with TT alleles (SEQ ID NO:1) at that locus pimecrolimus is selected as treatment, while for patients with CC alleles (SEQ ID NO:2) and CT alleles (SEQ ID NOS:1 and 2) a higher dose of pimecrolimus, pimecrolimus plus another anti-inflammatory drug or an anti-inflammatory drug without pimecrolimus is selected as treatment. Another embodiment provides that said treatment strategy is based upon a determination of the patient's *LTA* alleles. In a preferred embodiment, patients are tested for the presence of an ASN60THR *LTA* locus polymorphism to determine the treatment strategy. For patients with AA alleles (SEQ ID NO:3) or AC alleles (SEQ ID NOS:3 and 4) at that locus pimecrolimus is selected as treatment, while for patients with CC alleles (SEQ ID NO:4) a higher dose of pimecrolimus, pimecrolimus plus another anti-inflammatory drug or an anti-inflammatory drug without pimecrolimus is selected as treatment. The anti-inflammatory drug is preferably selected from the group consisting of hydrocortisone, cyclosporine, tacrolimus and sirolimus.

The disclosure provides a further method for determining a treatment strategy for a subject / patient affected by atopic dermatitis, psoriasis, asthma, inflammatory bowel disease, rheumatoid arthritis or other condition for which pimecrolimus or tacrolimus is indicated, based upon a determination of the patient's *CCR2* alleles. In one embodiment, the patient is tested for the presence of a VAL64ILE *CCR2* locus polymorphism to determine the appropriate treatment strategy. For a patient with GG allele (SEQ ID NO:5) at that locus tacrolimus is selected as treatment strategy, while for a patient with AG allele (SEQ ID NOS:5 and 6) a higher dose of tacrolimus, tacrolimus plus another anti-inflammatory drug or an anti-inflammatory drug without tacrolimus is selected as treatment strategy.. The anti-inflammatory drug is preferably selected from the group consisting of hydrocortisone, cyclosporine, pimecrolimus and sirolimus.

The disclosure also provides the use of pimecrolimus in the manufacture of a medicament for the treatment of inflammatory disease in a selected patient population, wherein the patient population is selected on the basis of the genotype of the patients at a genetic locus from the TNF gene cluster indicative of efficacy of pimecrolimus in treating inflammatory disease. In one embodiment, the genotype of the selected patient population comprises the presence of a (-1031) *TNF* polymorphism. Preferably, the selected patient population comprises TT alleles at that locus. In another embodiment, the selected patient population comprises an ASN60THR *LTA* locus polymorphism. Preferably, the selected patient population comprises AA alleles or AC alleles at that locus.

Further, the disclosure provides the use of tacrolimus in the manufacture of a medicament for the treatment of inflammatory disease in a selected patient population, wherein the patient population is selected on the basis of the genotype of the patients at the *CCR2* genetic locus indicative of efficacy of tacrolimus in treating inflammatory disease. In one embodiment, the selected patient population comprises the VAL64ILE *CCR2* locus polymorphism. Preferably, said patient population comprise GG alleles at that locus.

The invention thus provides a way of improving the efficacy of topical macrolactams, particularly pimecrolimus, by identifying biomarkers of efficacy in the subjects to be treated and then targeting appropriate macrolactam formulations to those who are best able to respond and benefit from the treatment. This invention is defined in the appended claims.

### BRIEF DESCRIPTION OF THE DRAWINGS

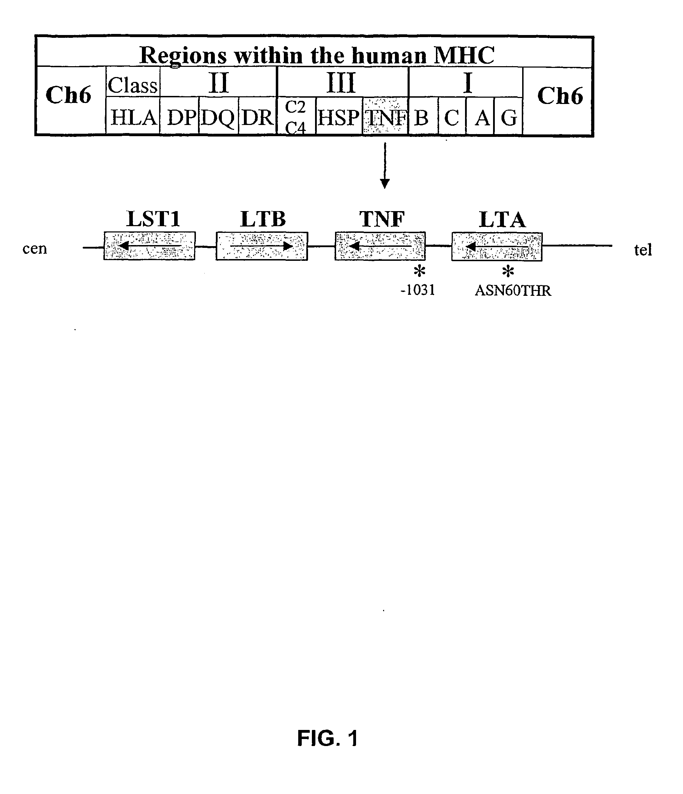

FIG. 1 is a diagrammatic representation of the major histocompatability complex (MHC) on chromosome (Ch) 6 showing the placement of LTA (gene coding for lymphotoxin alpha) and TNF (gene coding for tumor necrosis factor alpha; TNF-α) within the MHC. Shown in greater detail below is the region of 6p21.3 that contains the TNF gene cluster. * denotes polymorphisms, which are roughly 2.5 kb apart, discussed herein. This figure is modified from Field M, Q J Med 94: 237-246 (2001).

### DESCRIPTION OF THE PREFERRED EMBODIMENTS

The efficacy of treatment of atopic dermatitis by pimecrolimus cream 1%, but not treatment by tacrolimus ointment 0.03%, is associated with polymorphic markers in the *TNF* cluster on 6p21.3. This discovery provides a way to use genetic polymorphisms that associate with the efficacy of pimecrolimus treatment in determining the most effective treatment.

The genetic locus was identified in a Phase IIIb trial that was designed as a head to head comparison of pimecrolimus cream 1% and tacrolimus ointment 0.03%. The six-week investigator-blinded trial ofpediatric subjects with moderate atopic dermatitis explored the incidence of local application site reactions induced by the two topical macrolactams, as well as their efficacy, safety and cosmetic acceptability. Blood samples for pharmacogenetic analysis were collected from consenting participants in the clinical trial, to define a genetic basis for response to either treatment.

Among those genes selected for pharmacogenomic analysis were *LTA,* which codes for lymphotoxin alpha (LTα), and *TNF,* which codes for tumor necrosis factor alpha (TNF-α). The *LTA* and *TNF* genes are tandemly located in the gene-rich MHCIII region of 6p21.3 (see, FIG. 1). Both genes contain multiple polymorphisms, some of which have been well-characterized, as reviewed by Field M, Q J Med 94: 237-246 (2001). Both the *LTA* and the *TNF* gene products are cytokines involved in inflammatory processes. Both cytokines signal through TNF-α receptors, which are expressed in many different tissues, including the skin. Rulls SR & Sedgwick JD, Am J Hum Genet 65: 294-301 (1999). Furthermore, both *LTA* and *TNF* have been implicated in the pathogenesis of atopic disease. Polymorphisms in both these genes are associated with atopy, particularly asthma. Trabetti E et al., J Med Genet 36: 323-5 (1999); Izakovicaova Holla L et al., Clin Exp Allergy 9: 1418-23 (2001); Castro J et al., J Investig Allergol Clin Immunol 3:149-5 (2000); Li Kam Wa TC et al., Clin Exp Allergy 29: 1204-8 (1999); and Zhu S et al. Am J Respir Crit Care Med 161: 1655 - 9 (2000). TNF-α has been extensively studied for its role in dermal inflammation. Both dermal mast cells and keratinocytes produce TNF-α. While expression of TNF-α is constitutive in the skin, the level of expression can be further induced by a variety of stimuli. TNF-α levels are elevated in skin lesions of psoriasis, and several reports have suggested that drugs that modulate TNF-α function are effective in the treatment of psoriasis, as reviewed by LaDuca JR & Gaspari AA, Dermatol Clin 19: 617-635 (2001) and Mease PJ, Ann Rheum Dis 61: 298-304 (2002). Though psoriasis is distinct from atopic dermatitis, the expression of both diseases is influenced by genes that modulate dermal inflammation. Cookson WO et al., Nature Genetics 27: 372-373 (2001).

In the clinical trial and subsequent pharmacogenomics analysis, an association between pimecrolimus efficacy and the (-1031) *TNF* polymorphism was found in pediatric subjects (ages 2-17 years) with atopic dermatitis who were treated topically with the cream formulation of pimecrolimus. The polymorphism that associated with pimecrolimus efficacy is located -1031 from the transcription start site of the gene and changes the wild-type T to a C. The only subjects that experienced efficacy in this trial had a TT genotype at (-1031) *TNF* (SEQ ID NO:1). No patient with a C allele (CC (SEQ ID NO:2) or CT(SEQ ID NOS:1 and 2)) at that locus responded to pimecrolimus. This association did not hold for tacrolimus.

For a second SNP in the same chromosomal region, ASN60THR *LTA,* the data also suggested a trend toward significance.

These associations can reasonably be expected to hold for all age groups and for subjects with other inflammatory skin diseases. Moreover, the efficacy of pimecrolimus can reasonably be predicted when the compound is formulated as a tablet or aerosol. The method can be used for vertebrate subjects, particularly mammalian subjects and more particularly for human subjects.

Individuals carrying polymorphic alleles of interest (*i.e*., in genes from the *TNF* cluster, such as *TNF* and *LTA)* may be detected at the DNA, the RNA, or the protein level using a variety of techniques that are well known in the art. Strategies for identification and detection are described in *e.g*. EP 730,663, EP 717,113, and PCT US97/02102. The methods may involve the detection of pre-characterized polymorphisms, where the genotyping location and nature of polymorphic forms present at a site have already been determined (see, discussion above regarding *LTA* and *TNF* genes). The availability of this information allows sets of probes to be designed for specific identification of the known polymorphic forms. The identification of alleles containing single nucleotide polymorphisms may involve the amplification of DNA from target samples. This can be accomplished by *e.g.,* PCR. See generally PCR Technology: Principles and Applications for DNA Amplification, (ed. Erlich, Freeman Press, NY, N.Y., 1992); PCR Protocols: A Guide to Methods and Applications (eds. Innis, et al., Academic Press, San Diego, Calif., 1990). The detection of polymorphisms in specific DNA sequences, can be accomplished by a variety of methods including, but not limited to, restriction-fragment-length-polymorphism detection based on allele-specific restriction-endonuclease cleavage (Kan & Dozy, Lancet II:910-912 (1978)), hybridization with allele-specific oligonucleotide probes (Wallace et al, Nucl. Acids Res. 6:3543-3557 (1978)), including immobilized oligonucleotides (Saiki et al., Proc. Natl. Acad. Sci. USA, 86:6230-6234 (1969)) or oligonucleotide arrays (Maskos & Southern, Nucl. Acids Res. 21:2269-2270 (1993)), allele-specific PCR (Newton et al., Nucl. Acids Res. 17:2503-2516 (1989)), mismatch-repair detection (MRD) (Faham & Cox, Genome Res. 5:474-482 (1995)), binding of MutS protein (Wagner et al., Nuc/. Acids Res. 23:3944-3948 (1995), denaturing-gradient gel electrophoresis (DGGE) (Fisher & Lerman, Proc. Natl. Acad. Sci. U.S.A. 80:1579-1583 (1983)), single-strand-conformation-- polymorphism detection (Orita et al., Genomics 5:874-879 (1983)), RNAse cleavage at mismatched base-pairs (Myers et al., Science 230:1242 (1985)), chemical (Cotton et al., Proc. Natl. Acad. Sci. U.S.A., 8Z:4397-4401 (1988)) or enzymatic (Youil et al., Proc. Natl. Acad. Sci. U.S.A. 92:87-91 (1995)) cleavage of heteroduplex DNA, methods based on allele specific primer extension (Syvanen et al., Genomics 8:684-692 (1990)), genetic bit analysis (GBA) (Nikiforov et al., Nucl. Acids Res. 22:4167-4175 (1994)), the oligonucleotide-ligation assay (OLA) (Landegren et al., Science 241:1077 (1988)), the allele-specific ligation chain reaction (LCR) (Barrany, Proc. Natl. Acad. Sci. U.S.A. 88:189-193 (1991)), gap-LCR (Abravaya et al., Nucl. Acids Res. 23:675-682 (1995)), radioactive and/or fluorescent DNA sequencing using standard procedures well known in the art, and peptide nucleic acid (PNA) assays (Orum et al., Nucl. Acids Res. 21:5332-5356 (1993); Thiede et al., Nucl. Acids Res. 24:983-984 (1996)). Additional guidance is provided by Sambrook J et al., Molecular Cloning: A Laboratory Manual, Third Edition (Cold Spring Harbor Press, Cold Spring Harbor, 2000).

Levels of TNF-α mRNA or protein can be determined by methods known to those of skill in the art. See, U.S. Pat. Nos. 6,566,501; 6,541,620; and 6,537,540. A discussion of the levels of the level of tumor necrosis factor in TNF-associated disorders is provided in U.S. Pat. No. 6,537,540.

As used herein, the level of TNF-α mRNA (SEQ ID NO:7) in the sample is determined to be "high" when the ratio of TNF-α mRNA to β-actin expression (SEQ ID NO:9; see, Actor JK et al., Comb Chem High Throughput Screen 3(4):343-51 (August 2000)) or GAPDH expression (see, Anderson GD et al., J Clin Invest. 97(11):2672-9 (June 1, 1996)) in the sample is about twice as high or higher than the ratio of TNF-α mRNA to β-actin expression, as compared with a sample from an individual (or preferably a population of individuals) who does not have an inflammatory skin condition. As used herein, the level of TNF-a mRNA in the sample is determined to be "low or normal" when the ratio of TNF-α mRNA to β-actin expression in the sample is about equal to or less than the ratio of TNF-α mRNA to β-actin expression, as compared with a sample from an individual (or preferably, a population of individuals) who does not have an inflammatory skin condition. For guidance as to which TNF-α mRNA and protein levels tissue are generally expected to be "low", "normal" or "high", see, Van Deventer SJH, Gut 40: 443-8 (1997); McAlindon ME & Mahida YR, Aliment Pharmacol Ther 10(suppl 2): 72-4 (1996); Reimund J-M et al., J Clin Immunol 16: 144-50 (1996); Reinecker H-C et al., Clin Exp Immunol 94: 174-81 (1993); Murch SH et al., Gut. 34: 1705-9 (1993) and Grom AA et al., Arthritis Rheum 39: 1703-1710 (1996). Alternatively, one of skill in the art can define an average or expected level of TNF-α mRNA expression in a population, *e.g*., by performing the assay in ten or more control subjects (such as by methods shown in the EXAMPLE) and obtaining the average and standard deviation.

Samples for the determination of genetic polymorphisms, *TNF* mRNA levels (SEQ ID NO:7) or TNF protein levels (SEQ ID NO:8) can be collected from subjects by any appropriate method know to those of skill in the medical art. Samples can be fluid samples, such as from blood, mucus, lymphatic fluid, synovial fluid, cerebrospinal fluid, saliva, amniotic fluid, amniotic cord blood, urine, vaginal fluid and semen or other bodily fluid. Alternatively, samples can be tissue samples, such as from biopsy samples, homogenate, lysate or extract prepared from a whole organism or a subset of its tissues, cells or component parts, or a fraction or portion thereof. A sample can also be a medium, such as a nutrient broth or gel in which an organism has been propagated, which contains cellular components, such as proteins or nucleic acid molecules.

Guidance for the use of pimecrolimus cream 1 % is provided by Novartis Pharmaceuticals Corp., Elidel® Prescribing Information, T2003-01, 89014902, 492573/1 US (Rev. April 2003). Additional guidance for the use ofpimecrolimus is provided by Nghiem P et al., J Am Acad Dermatol 46: 228-241 (2002) and references cited therein and in U.S. Pat. Nos. 5,912,238; 6,352,998 and 6,423,722.

Guidance for the use of tacrolimus ointment (0.03% or 0.1%) is provided by Fujisawa Healthcare Inc., Protopic® Prescribing Information, 45670 (December 2000). Guidance for the use of Prograf® (oral capsule or injectable) is provided by Fujisawa Healthcare Inc., Prograf® Prescribing Information, ZL40306 and Fujisawa Healthcare Inc., Prograf® Product Monograph. Additional guidance for the use of tacrolimus is provided by Nghiem P et al., J Am Acad Dermatol 46: 228-241 (2002) and references cited therein.

As used herein, a "high dose ofpimecrolimus" is a dose substantially higher than the recommended dose of pimecrolimus. For example, for adults and children over two years of age, a thin layer of pimecrolimus cream 1% is applied to the affected area twice times a day, then rubbed in gently. A dose substantially higher than the recommended dose may be the application four times a day. Alternatively, a dose substantially higher than the recommended dose may be the application twice a day of a pimecrolimus formulation with a concentration of 2% or higher, if such be made available. Likewise, a "high dose of tacrolimus" is a dose substantially higher than the recommended dose of tacrolimus.

The use of other immunosuppressants, such as hydrocortisone, cyclosporine or sirolimus (rapamycin), in dermatology generally and in particular for atopic dermatitis, is well-known to those of skill in the medical arts. See, Marsland AM & Griffiths CE, Eur J Dermatol. 2(6):618-22 (November-December 2002) and Nghiem P et al., J Am Acad Dermatol 46: 228-241 (2002).

The diagnosis of atopic dermatitis is well-known to those of skill in the medical arts. See, Cookson WO et al., Nature Genetics 27: 372-373 (2001); Nghiem P et al., J Am Acad Dermatol 46: 228-241 (2002) and references cited therein.

*Single Nucleotide Polymorphisms.* Sequence variation in the human genome consists primarily of single nucleotide polymorphisms ("SNPs") with the remainder of the sequence variations being short tandem repeats (including micro-satellites), long tandem repeats (mini-satellite) and other insertions and deletions. A SNP is a position at which two alternative bases occur at appreciable frequency (*i.e.* >1%) in the human population. A SNP is said to be "allelic" in that due to the existence of the polymorphism, some members of a species may have the unmutated sequence (i.e., the original "allele") whereas other members may have a mutated sequence (i.e., the variant or mutant allele). In the simplest case, only one mutated sequence may exist, and the polymorphism is said to be diallelic. The occurrence of alternative mutations can give rise to triallelic polymorphisms, *etc.* SNPs are widespread throughout the genome and SNPs that alter the function of a gene may be direct contributors to phenotypic variation. Due to their prevalence and widespread nature, SNPs have potential to be important tools for locating genes that are involved in human disease conditions, *see e.g.,* Wang et al., Science 280: 1077-1082 (1998), which discloses a pilot study in which 2,227 SNPs were mapped over a 2.3 megabase region of DNA.

An association between a single nucleotide polymorphisms and a particular phenotype does not indicate or require that the SNP is causative of the phenotype. Instead, such an association may indicate only that the SNP is located near the site on the genome where the determining factors for the phenotype exist and therefore is more likely to be found in association with these determining factors and thus with the phenotype of interest. Thus, a SNP may be in linkage disequilibrium (LD) with the 'true' functional variant. LD, also known as allelic association exists when alleles at two distinct locations of the genome are more highly associated than expected.

Thus a SNP may serve as a marker that has value by virtue of its proximity to a mutation that causes a particular phenotype.

SNPs that are associated with disease may also have a direct effect on the function of the gene in which they are located. A sequence variant may result in an amino acid change or may alter exon-intron splicing, thereby directly modifying the relevant protein, or it may exist in a regulatory region, altering the cycle of expression or the stability of the mRNA, see Nowotny P Current Opinions in Neurobiology 11:637-641 (2001).

It is increasingly clear that the risk of developing many common disorders and the metabolism of medications used to treat these conditions are substantially influenced by underlying genomic variations, although the effects of any one variant might be small.

Therefore, an association between a SNP and a clinical phenotype suggests, (1) the SNP is functionally responsible for the phenotype or, (2) there are other mutations near the location of the SNP on the genome that cause the phenotype. The 2^{nd} possibility is based on the biology of inheritance. Large pieces of DNA are inherited and markers in close proximity to each other may not have been recombined in individuals that are unrelated for many generations, *i.e*., the markers are in linkage disequlibrium (LD).

*Identification and characterization of SNPs.* Many different techniques can be used to identify and characterize SNPs, including single-strand conformation polymorphism analysis, heteroduplex analysis by denaturing high-performance liquid chromatography (DHPLC), direct DNA sequencing and computational methods, *see* Shi MM, Clin Chem 47:164-172 (2001). Thanks to the wealth of sequence information in public databases, computational tools can be used to identify SNPs *in silico* by aligning independently submitted sequences for a given gene (either cDNA or genomic sequences). Comparison of SNPs obtained experimentally and by *in silico* methods showed that 55% of candidate SNPs found by SNPFinder(http://Ipgws.nci.nih.gov:82/perl/snp/snp_cgi.pl) have also been discovered experimentally, *see,* Cox et al. Hum Mutat 17:141-150 (2001). However, these *in silico* methods could only find 27% of true SNPs.

The most common SNP typing methods currently include hybridization, primer extension and cleavage methods. Each of these methods must be connected to an appropriate detection system. Detection technologies include fluorescent polarization, (see Chan X et al. Genome Res 9:492-499 (1999)), luminometric detection of pyrophosphate release (pyrosequencing), (see Ahmadiian A et al., Anal Biochem 280:103-10 (2000)), fluorescence resonance energy transfer (FRET)-based cleavage assays, DHPLC, and mass spectrometry, (see Shi MM, Clin Chem 47:164-172 (2001) and U.S. Pat. No. 6,300,076 Bl). Other methods of detecting and characterizing SNPs are those disclosed in U.S. Patents No. 6,297,018 B1 and 6,300,063 B1.

In a particularly preferred embodiment the detection of the polymorphism can be accomplished by means of so called INVADER™ technology (available from Third Wave Technologies Inc. Madison, Wis.). In this assay, a specific upstream "invader" oligonucleotide and a partially overlapping downstream probe together form a specific structure when bound to complementary DNA template. This structure is recognized and cut at a specific site by the Cleavase enzyme, and this results in the release of the 5' flap of the probe oligonucleotide. This fragment then serves as the "invader" oligonucleotide with respect to synthetic secondary targets and secondary fluorescently labeled signal probes contained in the reaction mixture. This results in specific cleavage of the secondary signal probes by the Cleavase enzyme. Fluorescence signal is generated when this secondary probe , labeled with dye molecules capable of fluorescence resonance energy transfer, is cleaved. Cleavases have stringent requirements relative to the structure formed by the overlapping DNA sequences or flaps and can, therefore, be used to specifically detect single base pair mismatches immediately upstream of the cleavage site on the downstream DNA strand. See Ryan D et al. Molecular Diagnosis 4(2):135-144 (1999) and Lyamichev V et al. Nature Biotechnology 17:292-296 (1999), see also US Patents 5,846,717 and 6,001,567.

In some embodiments, a composition contains two or more differently labeled genotyping oligonucleotides for simultaneously probing the identity of nucleotides at two or more polymorphic sites. It is also contemplated that primer compositions may contain two or more sets of allele-specific primer pairs to allow simultaneous targeting and amplification of two or more regions containing a polymorphic site.

Genotyping oligonucleotides may also be immobilized on or synthesized on a solid surface such as a microchip, bead. or glass slide (see, *e.g*., WO 98/20020 and WO 98/20019). Such immobilized genotyping oligonucleotides may be used in a variety of polymorphism detection assays, including but not limited to probe hybridization and polymerase extension assays. Immobilized genotyping oligonucleotides may comprise an ordered array of oligonucleotides designed to rapidly screen a DNA sample for polymorphisms in multiple genes at the same time.

An allele-specific oligonucleotide primer has a 3' terminal nucleotide, or preferably a 3' penultimate nucleotide, that is complementary to only one nucleotide of a particular SNP, thereby acting as a primer for polymerase-mediated extension only if the allele containing that nucleotide is present. Allele-specific oligonucleotide primers hybridizing to either the coding or noncoding strand are contemplated. An ASO primer for detecting gene polymorphisms could be developed using techniques known to those of skill in the art.

Other genotyping oligonucleotides hybridize to a target region located one to several nucleotides downstream of one of the novel polymorphic sites identified herein. Such oligonucleotides are useful in polymerase-mediated primer extension methods for detecting one of the novel polymorphisms described herein and therefore such genotyping oligonucleotides are referred to herein as "primer-extension oligonucleotides". In a preferred embodiment, the 3'-terminus of a primer-extension oligonucleotide is a deoxynucleotide complementary to the nucleotide located immediately adjacent to the polymorphic site.

In another embodiment, the disclosure provides a kit comprising at least two genotyping oligonucleotides packaged in separate containers. The kit may also contain other components such as hybridization buffer (where the oligonucleotides are to be used as a probe) packaged in a separate container. Alternatively, where the oligonucleotides are to be used to amplify a target region, the kit may contain, packaged in separate containers, a polymerase and a reaction buffer optimized for primer extension mediated by the polymerase, such as PCR.

The above described oligonucleotide compositions and kits are useful in methods for genotyping and/or haplotyping the gene in an individual. As used herein, the terms " genotype" and " haplotype" mean the genotype or haplotype containing the nucleotide pair or nucleotide, respectively, that is present at one or more of the novel polymorphic sites described herein and may optionally also include the nucleotide pair or nucleotide present at one or more additional polymorphic sites in the gene. The additional polymorphic sites may be currently known polymorphic sites or sites that are subsequently discovered.

One embodiment of the genotyping method involves isolating from the individual a nucleic acid mixture comprising the two copies of the gene, or a fragment thereof, that are present in the individual, and determining the identity of the nucleotide pair at one or more of the polymorphic sites in the two copies to assign a genotype to the individual. As will be readily understood by the skilled artisan, the two "copies" of a gene in an individual may be the same allele or may be different alleles. In a particularly preferred embodiment, the genotyping method comprises determining the identity of the nucleotide pair at each polymorphic site.

Typically, the nucleic acid mixture is isolated from a biological sample taken from the individual, such as a blood sample or tissue sample. Suitable tissue samples include whole blood, semen, saliva, tears, urine, fecal material, sweat, buccal smears, skin and hair. The nucleic acid mixture may be comprised of genomic DNA, mRNA, or cDNA and, in the latter two cases, the biological sample must be obtained from an organ in which the gene is expressed. Furthermore it will be understood by the skilled artisan that mRNA or cDNA preparations would not be used to detect polymorphisms located in introns or in 5' and 3' nontranscribed regions. If a gene fragment is isolated, it must contain the polymorphic site(s) to be genotyped.

One embodiment of the haplotyping method comprises isolating from the individual a nucleic acid molecule containing only one of the two copies of the gene, or a fragment thereof, that is present in the individual and determining in that copy the identity of the nucleotide at one or more of the polymorphic sites in that copy to assign a haplotype to the individual. The nucleic acid may be isolated using any method capable of separating the two copies of the gene or fragment, including but not limited to, one of the methods described above for preparing isogenes, with targeted *in vivo* cloning being the preferred approach. As will be readily appreciated by those skilled in the art, any individual clone will only provide haplotype information on one of the two gene copies present in an individual. If haplotype information is desired for the individual's other copy, additional clones will need to be examined. Typically, at least five clones should be examined to have more than a 90% probability of haplotyping both copies of the gene in an individual. In a particularly preferred embodiment, the nucleotide at each of polymorphic site is identified.

In a preferred embodiment, a haplotype pair is determined for an individual by identifying the phased sequence of nucleotides at one or more of the polymorphic sites in each copy of the gene that is present in the individual. In a particularly preferred embodiment, the haplotyping method comprises identifying the phased sequence of nucleotides at each polymorphic site in each copy of the gene. When haplotyping both copies of the gene, the identifying step is preferably performed with each copy of the gene being placed in separate containers. However, it is also envisioned that if the two copies are labeled with different tags, or are otherwise separately distinguishable or identifiable, it could be possible in some cases to perform the method in the same container. For example, if first and second copies of the gene are labeled with different first and second fluorescent dyes, respectively, and an allele-specific oligonucleotide labeled with yet a third different fluorescent dye is used to assay the polymorphic site(s), then detecting a combination of the first and third dyes would identify the polymorphism in the first gene copy while detecting a combination of the second and third dyes would identify the polymorphism in the second gene copy.

In both the genotyping and haplotyping methods, the identity of a nucleotide (or nucleotide pair) at a polymorphic site(s) may be determined by amplifying a target region(s) containing the polymorphic site(s) directly from one or both copies of the gene, or fragment thereof, and the sequence of the amplified region(s) determined by conventional methods. It will be readily appreciated by the skilled artisan that only one nucleotide will be detected at a polymorphic site in individuals who are homozygous at that site, while two different nucleotides will be detected if the individual is heterozygous for that site. The polymorphism may be identified directly, known as positive-type identification, or by inference, referred to as negative-type identification. For example, where a SNP is known to be guanine and cytosine in a reference population, a site may be positively determined to be either guanine or cytosine for ail individual homozygous at that site, or both guanine and cytosine, if the individual is heterozygous at that site. Alternatively, the site may be negatively determined to be not guanine (and thus cytosine/cytosine) or not cytosine (and thus guanine/guanine).

In addition, the identity of the allele(s) present at any of the novel polymorphic sites described herein may be indirectly determined by genotyping a polymorphic site not disclosed herein that is in linkage disequilibrium with the polymorphic site that is of interest. Two sites are said to be in linkage disequilibrium if the presence of a particular variant at one site enhances the predictability of another variant at the second site (*see,* Stevens JC, Mol Diag 4:309-317 (1999)). Polymorphic sites in linkage disequilibrium with the presently disclosed polymorphic sites may be located in regions of the gene or in other genomic regions not examined herein. Genotyping of a polymorphic site in linkage disequilibrium with the novel polymorphic sites described herein may be performed by, but is not limited to, any of the above-mentioned methods for detecting the identity of the allele at a polymorphic site.

The target region(s) may be amplified using any oligonucleotide-directed amplification method, including but not limited to polymerase chain reaction (PCR) (U.S. Pat. No. 4,965,188), ligase chain reaction (LCR) (Barany et al., Proc Natl Acad Sci USA 88:189-193 (1991); PCT patent application WO 90/01069), and oligonucleotide ligation assay (OLA) (Landegren et al., Science 241:1077-1080 (1988)). Oligonucleotides useful as primers or probes in such methods should specifically hybridize to a region of the nucleic acid that contains or is adjacent to the polymorphic site. Typically, the oligonucleotides are between 10 and 35 nucleotides in length and preferably, between 15 and 30 nucleotides in length. Most preferably, the oligonucleotides are 20 to 25 nucleotides long. The exact length of the oligonucleotide will depend on many factors that are routinely considered and practiced by the skilled artisan.

Other known nucleic acid amplification procedures may be used to amplify the target region including transcription-based amplification systems (U.S. Pat. No. 5,130,238; EP 329,822; U.S. Pat. No. 5,169,766, WO 89/06700) and isothermal methods (Walker et al., Proc Natl Acad Sci USA 89:392-396 (1992)).

A polymorphism in the target region may also be assayed before or after amplification using one of several hybridization-based methods known in the art. Typically, allele-specific oligonucleotides are utilized in performing such methods. The allele-specific oligonucleotides may be used as differently labeled probe pairs, with one member of the pair showing a perfect match to one variant of a target sequence and the other member showing a perfect match to a different variant. In some embodiments, more than one polymorphic site may be detected at once using a set of allele-specific oligonucleotides or oligonucleotide pairs. Preferably, the members of the set have melting temperatures within 5°C and more preferably within 2°C, of each other when hybridizing to each of the polymorphic sites being detected.

Hybridization of an allele-specific oligonucleotide to a target polynucleotide may be performed with both entities in solution or such hybridization may be performed when either the oligonucleotide or the target polynucleotide is covalently or noncovalently affixed to a solid support. Attachment may be mediated, for example, by antibody-antigen interactions, poly-L-Lys, streptavidin or avidin-biotin, salt bridges, hydrophobic interactions, chemical linkages, UV cross-linking baking, etc. Allele-specific oligonucleotides may be synthesized directly on the solid support or attached to the solid support subsequent to synthesis. Solid-supports suitable for use in detection methods include substrates made of silicon, glass, plastic, paper and the like, which may be formed, for example, into wells (as in 96-well plates), slides, sheets, membranes, fibers, chips, dishes, and beads. The solid support may be treated, coated or derivatized to facilitate the immobilization of the allele-specific oligonucleotide or target nucleic acid.

The genotype or haplotype for the gene of an individual may also be determined by hybridization of a nucleic sample containing one or both copies of the gene to nucleic acid arrays and subarrays such as described in WO 95/11995. The arrays would contain a battery of allele-specific oligonucleotides representing each of the polymorphic sites to be included in the genotype or haplotype.

The identity of polymorphisms may also be determined using a mismatch detection technique, including but not limited to the RNase protection method using riboprobes (Winter et al., Proc Natl Acad Sci USA 82:7575 (1985); Meyers et al., Science 230:1242 (1985)) and proteins which recognize nucleotide mismatches, such as the *E*. *coli* mutS protein (Modrich P. Ann Rev Genet 25:229-253 (1991)). Alternatively, variant alleles can be identified by single strand conformation polymorphism (SSCP) analysis (Orita et al., Genomics 5:874-879 (1989); Humphries et al., in Molecular Diagnosis of Genetic Diseases, R. Elles, ed., pp. 321-340 (1996)) or denaturing gradient gel electrophoresis (DGGE) (Wartell et al., Nucl Acids Res 18:2699-2706 (1990); Sheffield et al., Proc Natl Acad Sci USA 86:232-236 (1989)).

A polymerase-mediated primer extension method may also be used to identify the polymorphism(s). Several such methods have been described in the patent and scientific literature and include the "Genetic Bit Analysis" method (WO 92/15712) and the ligase / polymerase mediated genetic bit analysis (U.S. Pat. No. 5,679,524). Related methods are disclosed in WO 91/02087, WO 90/09455, WO 95/17676, U.S. Pat. Nos. 5,302,509 and 5,945,283. Extended primers containing a polymorphism may be detected by mass spectrometry as described in U.S. Pat. No. 5,605,798. Another primer extension method is allele-specific PCR (Ruafio et al., Nucl Acids Res 17:8392 (1989); Ruafio et al., Nucl Acids Res 19:6877-6882 (1991); WO 93/22456; Turki et al., J Clin Invest 95:1635-1641 (1995)). In addition, multiple polymorphic sites may be investigated by simultaneously amplifying multiple regions of the nucleic acid using sets of allele-specific primers as described in WO 89/10414.

In a preferred embodiment, the haplotype frequency data for each ethnogeographic group is examined to determine whether it is consistent with Hardy-Weinberg equilibrium. Hardy-Weinberg equilibrium (D.L. Hartl et al., Principles of Population Genomics, 3rd Ed. (Sinauer Associates, Sunderland, MA, 1997) postulates that the frequency of finding the haplotype pair *H*₁/*H*₂ is equal to *P_{H-W}* (*H*₁/*H*₂) = 2*p*(*H*₁) *p* (*H*₂) if *H*₁ ≠ *H*₂ and *P_{H-W}* (*H*₁/*H*₂) *= p* (*H*₁) *p* (*H*₂) if *H*₁ = *H*₂. A statistically significant difference between the observed and expected haplotype frequencies could be due to one or more factors including significant inbreeding in the population group, strong selective pressure on the gene, sampling bias, and/or errors in the genotyping process. If large deviations from Hardy-Weinberg equilibrium are observed in an ethnogeographic group, the number of individuals in that group can be increased to see if the deviation is due to a sampling bias. If a larger sample size does not reduce the difference between observed and expected haplotype pair frequencies, then one may wish to consider haplotyping the individual using a direct haplotyping method such as, for example, CLASPER System™ technology (U.S. Pat. No. 5,866,404), SMD, or allele-specific long-range PCR (Michalotos-Beloin et al., Nucl Acids Res 24:4841-4843 (1996)).

In one embodiment of this method for predicting a haplotype pair, the assigning step involves performing the following analysis. First, each of the possible haplotype pairs is compared to the haplotype pairs in the reference population. Generally, only one of the haplotype pairs in the reference population matches a possible haplotype pair and that pair is assigned to the individual. Occasionally, only one haplotype represented in the reference haplotype pairs is consistent with a possible haplotype pair for an individual, and in such cases the individual is assigned a haplotype pair containing this known haplotype and a new haplotype derived by subtracting the known haplotype from the possible haplotype pair. In rare cases, either no haplotypes in the reference population are consistent with the possible haplotype pairs, or alternatively, multiple reference haplotype pairs are consistent with the possible haplotype pairs. In such cases, the individual is preferably haplotyped using a direct molecular haplotyping method such as, for example, CLASPER System™ technology (U.S. Pat. No. 5,866,404), SMD, or allele-specific long-range PCR (Michalotos-Beloin et al., Nucl Acids Res 24:4841-4843 (1996)).

The disclosure also provides a method for determining the frequency of a genotype or haplotype in a population. The method comprises determining the genotype or the haplotype pair for the gene that is present in each member of the population, wherein the genotype or haplotype comprises the nucleotide pair or nucleotide detected at one or more of the polymorphic sites in the gene, and calculating the frequency any particular genotype or haplotype is found in the population. The population may be a reference population, a family population, a same sex population, a population group, a trait population (*e.g*., a group of individuals exhibiting a trait of interest such as a medical condition or response to a therapeutic treatment).

In another aspect of the disclosure, frequency data for genotypes and/or haplotypes found in a reference population are used in a method for identifying an association between a trait and a genotype or a haplotype. The trait may be any detectable phenotype, including but not limited to susceptibility to a disease or response to a treatment. The method involves obtaining data on the frequency of the genotype(s) or haplotype(s) of interest in a reference population as well as in a population exhibiting the trait. Frequency data for one or both of the reference and trait populations may be obtained by genotyping or haplotyping each individual in the populations using one of the methods described above. The haplotypes for the trait population may be determined directly or, alternatively, by the predictive genotype to haplotype approach described above.

In another embodiment, the frequency data for the reference and/or trait populations is obtained by accessing previously determined frequency data, which may be in written or electronic form. For example, the frequency data may be present in a database that is accessible by a computer. Once the frequency data is obtained, the frequencies of the genotype(s) or haplotype(s) of interest in the reference and trait populations are compared. In a preferred embodiment, the frequencies of all genotypes and/or haplotypes observed in the populations are compared. If a particular genotype or haplotype for the gene is more frequent in the trait population than in the reference population at a statistically significant amount, then the trait is predicted to be associated with that genotype or haplotype.

In a preferred embodiment statistical analysis is performed by the use of standard ANOVA tests with a Bonferoni correction and/or a bootstrapping method that simulates the genotype phenotype correlation many times and calculates a significance value. When many polymorphisms are being analyzed a correction to factor may be performed to correct for a significant association that might be found by chance. For statistical methods see: Statistical Methods in Biology, 3rd edition, Bailey NTJ, (Cambridge Univ. Press, 1997); Introduction to Computational Biology, Waterman MS (CRC Press, 2000) and Bioinformatics, Baxevanis AD & Ouellette BFF editors (John Wiley & Sons, Inc., 2001).

In a preferred embodiment of the method, the trait of interest is a clinical response exhibited by a patient to some therapeutic treatment, for example, response to a drug targeting or response to a therapeutic treatment for a medical condition.

In another embodiment, a detectable genotype or haplotype that is in linkage disequilibrium with the genotype or haplotype of interest may be used as a surrogate marker. A genotype that is in linkage disequilibrium with a genotype may be discovered by determining if a particular genotype or haplotype for the gene is more frequent in the population that also demonstrates the potential surrogate marker genotype than in the reference population at a statistically significant amount, then the marker genotype is predicted to be associated with that genotype or haplotype and then can be used as a surrogate marker in place of the genotype.

*Definitions.* As used herein, "medical condition" includes but is not limited to any condition or disease manifested as one or more physical and/or psychological symptoms for which treatment is desirable, and includes previously and newly identified diseases and other disorders.

As used herein, the term "clinical response" means any or all of the following: a quantitative measure of the response, no response, and adverse response (i.e., side effects).

In order to deduce a correlation between clinical response to a treatment and a genotype or haplotype, data is obtained on the clinical responses exhibited by a population of individuals who received the treatment, hereinafter the "clinical population". This clinical data may be obtained by analyzing the results of a clinical trial that has already been run and/or the clinical data may be obtained by designing and carrying out one or more new clinical trials.

As used herein, the term "clinical trial" means any research study designed to collect clinical data on responses to a particular treatment, and includes but is not limited to phase I, phase II and phase III clinical trials. Standard methods are used to define the patient population and to enroll subjects.

It is preferred that the individuals included in the clinical population have been graded for the existence of the medical condition of interest. This grading of potential patients could employ a standard physical exam or one or more lab tests. Alternatively, grading of patients could use haplotyping for situations where there is a strong correlation between haplotype pair and disease susceptibility or severity.

The therapeutic treatment of interest is administered to each individual in the trial population and each individual's response to the treatment is measured using one or more predetermined criteria. It is contemplated that in many cases, the trial population will exhibit a range of responses and that the investigator will choose the number of responder groups (*e.g*., low, medium, high) made up by the various responses. In addition, the gene for each individual in the trial population is genotyped and/or haplotyped, which may be done before or after administering the treatment.

After both the clinical and polymorphism data have been obtained, correlations between individual response and genotype or haplotype content are created. Correlations may be produced in several ways. In one method, individuals are grouped by their genotype or haplotype (or haplotype pair) (also referred to as a polymorphism group), and then the averages and standard deviations of clinical responses exhibited by the members of each polymorphism group are calculated.

These results are then analyzed to determine if any observed variation in clinical response between polymorphism groups is statistically significant. Statistical analysis methods which may be used are described in L.D. Fisher & G. vanBelle, Biostatistics: A Methodology for the Health Sciences (Wiley-Interscience, New York, 1993). This analysis may also include a regression calculation of which polymorphic sites in the gene give the most significant contribution to the differences in phenotype.

A second method for finding correlations between haplotype content and clinical responses uses predictive models based on error-minimizing optimization algorithms. One of many possible optimization algorithms is a genetic algorithm (R. Judson, "Genetic Algorithms and Their Uses in Chemistry" in Reviews in Computational Chemistry, Vol. 10, pp. 1- 73, K.B. Lipkowitz & D.B. Boyd, eds. (VCH Publishers, New York, 1997). Simulated annealing (Press et al., "Numerical Recipes in C: The Art of Scientific Computing", Cambridge University Press (Cambridge) 1992, Ch. 10), neural networks (E. Rich and K. Knight, "Artificial Intelligence", 2nd Edition (McGraw-Hill, New York, 1991, Ch. 18), standard gradient descent methods (Press *et al., supra* Ch. 10), or other global or local optimization approaches (see discussion in Judson, supra) could also be used.

Correlations may also be analyzed using analysis of variation (ANOVA) techniques to determine how much of the variation in the clinical data is explained by different subsets of the polymorphic sites in the gene. ANOVA is used to test hypotheses about whether a response variable is caused by or correlated with one or more traits or variables that can be measured (Fisher & vanBelle, supra, Ch. 10).

From the analyses described above, a mathematical model may be readily constructed by the skilled artisan that predicts clinical response as a function of genotype or haplotype content.

The identification of an association between a clinical response and a genotype or haplotype (or haplotype pair) for the gene may be the basis for designing a diagnostic method to determine those individuals who will or will not respond to the treatment, or alternatively, will respond at a lower level and thus may require more treatment, *i.e*., a greater dose of a drug. The diagnostic method may take one of several forms: for example, a direct DNA test (*i.e*., genotyping or haplotyping one or more of the polymorphic sites in the gene), a serological test, or a physical exam measurement. The only requirement is that there be a good correlation between the diagnostic test results and the underlying genotype or haplotype that is in turn correlated with the clinical response. In a preferred embodiment, this diagnostic method uses the predictive haplotyping method described above.

A computer may implement any or all analytical and mathematical operations involved in practicing the methods of the present disclosure. In addition, the computer may execute a program that generates views (or screens) displayed on a display device and with which the user can interact to view and analyze large amounts of information relating to the gene and its genomic variation, including chromosome location, gene structure, and gene family, gene expression data, polymorphism data, genetic sequence data, and clinical data population data (*e.g*., data on ethnogeographic origin, clinical responses, genotypes, and haplotypes for one or more populations). The polymorphism data described herein may be stored as part of a relational database (*e.g*., an instance of an Oracle database or a set of ASCII flat files). These polymorphism data may be stored on the computer's hard drive or may, for example, be stored on a CD-ROM or on one or more other storage devices accessible by the computer. For example, the data may be stored on one or more databases in communication with the computer via a network.

In other embodiments, the disclosure provides methods, compositions, and kits for haplotyping and/or genotyping the gene in an individual. The compositions contain oligonucleotide probes and primers designed to specifically hybridize to one or more target regions containing, or that are adjacent to, a polymorphic site. The methods and compositions for establishing the genotype or haplotype of an individual at the novel polymorphic sites described herein are useful for studying the effect of the polymorphisms in the etiology of diseases affected by the expression and function of the protein, studying the efficacy of drugs targeting, predicting individual susceptibility to diseases affected by the expression and function of the protein and predicting individual responsiveness to drugs targeting the gene product.

In yet another embodiment, the disclosure provides a method for identifying an association between a genotype or haplotype and a trait. In preferred embodiments, the trait is susceptibility to a disease, severity of a disease, the staging of a disease or response to a drug. Such methods have applicability in developing diagnostic tests and therapeutic treatments for all pharmacogenetic applications where there is the potential for an association between a genotype and a treatment outcome including efficacy measurements, PK measurements and side effect measurements.

The disclosure also provides a computer system for storing and displaying polymorphism data determined for the gene. The computer system comprises a computer processing unit; a display; and a database containing the polymorphism data. The polymorphism data includes the polymorphisms, the genotypes and the haplotypes identified for the gene in a reference population. In a preferred embodiment, the computer system is capable of producing a display showing haplotypes organized according to their evolutionary relationships.

In another aspect, the disclosure provides SNP probes, which are useful in classifying people according to their types of genetic variation. The SNP probes are oligonucleotides, which can discriminate between alleles of a SNP nucleic acid in conventional allelic discrimination assays.

As used herein, a "SNP nucleic acid" is a nucleic acid sequence, which comprises a nucleotide that is variable within an otherwise identical nucleotide sequence between individuals or groups of individuals, thus, existing as alleles. Such SNP nucleic acids are preferably from about 15 to about 500 nucleotides in length. The SNP nucleic acids may be part of a chromosome, or they may be an exact copy of a part of a chromosome, *e.g*., by amplification of such a part of a chromosome through PCR or through cloning. The SNP nucleic acids are referred to hereafter simply as "SNPs". The SNP probes are oligonucleotides that are complementary to a SNP nucleic acid.

As used herein, the term "complementary" means exactly complementary throughout the length of the oligonucleotide in the Watson and Crick sense of the word.

In certain preferred embodiments, the oligonucleotides are complementary to one allele of the SNP nucleic acid, but not to any other allele of the SNP nucleic acid. Oligonucleotides according to this embodiment can discriminate between alleles of the SNP nucleic acid in various ways. For example, under stringent hybridization conditions, an oligonucleotide of appropriate length will hybridize to one allele of the SNP nucleic acid, but not to any other allele of the SNP nucleic acid. The oligonucleotide may be labeled by a radiolabel or a fluorescent label. Alternatively, an oligonucleotide of appropriate length can be used as a primer for PCR, wherein the 3' terminal nucleotide is complementary to one allele of the SNP nucleic acid, but not to any other allele. In this embodiment, the presence or absence of amplification by PCR determines the haplotype of the SNP nucleic acid.

Genomic and cDNA fragments comprise at least one novel polymorphic site identified herein and have a length of at least 10 nucleotides and may range up to the full length of the gene. Preferably, a fragment is between 100 and 3000 nucleotides in length, and more preferably between 200 and 2000 nucleotides in length, and most preferably between 500 and 1000 nucleotides in length.

In describing the polymorphic sites identified herein reference is made to the sense strand of the gene for convenience. However, as recognized by the skilled artisan, nucleic acid molecules containing the gene may be complementary double stranded molecules and thus reference to a particular site on the sense strand refers as well to the corresponding site on the complementary antisense strand. Thus, reference may be made to the same polymorphic site on either strand and an oligonucleotide may be designed to hybridize specifically to either strand at a target region containing the polymorphic site. Thus, the disclosure also includes single-stranded polynucleotides that are complementary to the sense strand of the genomic variants described herein.

In a preferred embodiment, such kit may further comprise a DNA sample collecting means.

In particular, the genotyping primer composition may comprise at least two sets of allele specific primer pairs. Preferably, the two genotyping oligonucleotides are packaged in separate containers.

It is to be understood that the methods described herein generally may further comprise the use of a kit. Generally, the methods may be performed *ex-vivo,* and such *ex-vivo* methods are specifically contemplated. Also, where a method may include steps that may be practiced on the human or animal body, methods that only comprise those steps which are not practiced on the human or animal body are specifically contemplated.

Effect(s) of the polymorphisms identified herein on expression of may be investigated by preparing recombinant cells and/or organisms, preferably recombinant animals, containing a polymorphic variant of the gene. As used herein, "expression" includes but is not limited to one or more of the following: transcription of the gene into precursor mRNA; splicing and other processing of the precursor mRNA to produce mature mRNA; mRNA stability; translation of the mature mRNA into protein (including codon usage and tRNA availability); and glycosylation and/or other modifications of the translation product, if required for proper expression and function.

To prepare a recombinant cell, the desired isogene may be introduced into the cell in a vector such that the isogene remains extrachromosomal. In such a situation, the gene will be expressed by the cell from the extrachromosomal location. In a preferred embodiment, the isogene is introduced into a cell in such a way that it recombines with the endogenous gene present in the cell. Such recombination requires the occurrence of a double recombination event, thereby resulting in the desired gene polymorphism. Vectors for the introduction of genes both for recombination and for extrachromosomal maintenance are known in the art, and any suitable vector or vector construct may be used. Methods such as electroporation, particle bombardment, calcium phosphate co-precipitation and viral transduction for introducing DNA into cells are known in the art; therefore, the choice of method may lie with the competence and preference of the skilled practitioner.

Recombinant organisms, i.e., transgenic animals, expressing a variant gene are prepared using standard procedures known in the art. Preferably, a construct comprising the variant gene is introduced into a nonhuman animal or an ancestor of the animal at an embryonic stage, i.e., the one-cell stage, or generally not later than about the eight-cell stage. Transgenic animals carrying the constructs can be made by several methods known to those having skill in the art. One method involves transfecting into the embryo a retrovirus constructed to contain one or more insulator elements, a gene or genes of interest, and other components known to those skilled in the art to provide a complete shuttle vector harboring the insulated gene(s) as a transgene, *see e.g.,* U.S. Pat. No. 5,610,053. Another method involves directly injecting a transgene into the embryo. A third method involves the use of embryonic stem cells.

Examples of animals, into which the isogenes may be introduced include, but are not limited to, mice, rats, other rodents, and nonhuman primates (see "The Introduction of Foreign Genes into Mice" and the cited references therein, In: Recombinant DNA, Eds. J .D. Watson, M. Gilman, J. Witkowski, & M. Zoller; W.H. Freeman and Company, New York, pages 254-272). Transgenic animals stably expressing a human isogene and producing human protein can be used as biological models for studying diseases related to abnormal expression and/or activity, and for screening and assaying various candidate drugs, compounds, and treatment regimens to reduce the symptoms or effects of these diseases.

In practicing the present disclosure, many conventional techniques in molecular biology, microbiology and recombinant DNA are used. These techniques are well-known and are explained in, *e.g., "*Current Protocols in Molecular Biology", Vols. I-III, Ausubel, Ed. (1997); Sambrook et al., "Molecular Cloning: A Laboratory Manual", 2nd Ed., Cold Spring Harbor Laboratory Press, Cold Spring Harbor, NY (1989); *"*DNA Cloning: A Practical Approach", Vols. I and II, Glover, Ed. (1985); *"*Oligonucleotide Synthesis", Gait, Ed. (1984); *"*Nucleic Acid Hybridization", Hames & Higgins, Eds. (1985); *"*Transcription and Translation", Hames & Higgins, Eds. (1984); *"*Animal Cell Culture", Freshney, Ed. (1986); *"*Immobilized Cells and Enzymes", IRL Press (1986); Perbal, "A Practical Guide to Molecular Cloning*";* the series, Methods in Enzymol., Academic Press, Inc. (1984); *"*Gene Transfer Vectors for Mammalian Cells", Miller and Calos, Eds., Cold Spring Harbor Laboratory, NY (1987); and Methods in Enzymology, Vols. 154 and 155, Wu & Grossman, and Wu, Eds., respectively.

The standard control levels of the gene expression product, thus determined in the different control groups, would then be compared with the measured level of an gene expression product in a given patient. This gene expression product could be the characteristic mRNA associated with that particular genotype group or the polypeptide gene expression product of that genotype group. The patient could then be classified or assigned to a particular genotype group based on how similar the measured levels were compared to the control levels for a given group.

As one of skill in the art will understand, there will be a certain degree of uncertainty involved in making this determination. Therefore, the standard deviations of the control group levels would be used to make a probabilistic determination and the methods would be applicable over a wide range of probability based genotype group determinations. Thus, for example and not by way of limitation, in one embodiment, if the measured level of the gene expression product falls within 2.5 standard deviations of the mean of any of the control groups, then that individual may be assigned to that genotype group. In another embodiment if the measured level of the gene expression product falls within 2.0 standard deviations of the mean of any of the control groups then that individual may be assigned to that genotype group. In still another embodiment, if the measured level of the gene expression product falls within 1.5 standard deviations of the mean of any of the control groups then that individual may be assigned to that genotype group. In yet another embodiment, if the measured level of the gene expression product is 1.0 or less standard deviations of the mean of any of the control groups levels then that individual may be assigned to that genotype group.

Thus this process will allow the determining, with various degrees of probability, which group a specific patient should be place in and such assignment to a genotype group would then determine the risk category into which the individual should be placed.

Methods to detect and measure mRNA levels and levels of polypeptide gene expression products are well known in the art and include the use of nucleotide microarrays and polypeptide detection methods involving mass spectrometers and/or antibody detection and quantification techniques. See also, Human Molecular Genetics, 2nd Edition. Tom Strachan & Andrew, Read (John Wiley and Sons, Inc. Publication, NY, 1999).

Furthermore, detection of the concentration of the polypeptide (protein) expression product of the gene in body fluids or tissues can be used to determine the presence or absence of the polymorphism, and the relative level of the polypeptide expression product can be used to determine if the polymorphism is present in a homozygous or heterozygous state and therefore the risk category of the individual.

As used herein, "medical condition" includes, but is not limited to, any condition or disease manifested as one or more physical and/or psychological symptoms for which treatment is desirable, and includes previously and newly-identified diseases and other disorders.

As used herein the term "polymorphism" shall mean any sequence variant present at a frequency of >1% in a population. The sequence variant may be present at a frequency significantly greater than 1% such as 5% or 10 % or more. Also, the term may be used to refer to the sequence variation observed in an individual at a polymorphic site. Polymorphisms include nucleotide substitutions, insertions, deletions and microsatellites and may, but need not, result in detectable differences in gene expression or protein function.

As used herein, the term "clinical response" means any or all of the following: a quantitative measure of the response, no response and adverse response, *i.e*., side effects.

As used herein the term "allele" shall mean a particular form of a gene or DNA sequence at a specific chromosomal location (locus).

As used herein, the term "genotype" shall mean an unphased 5' to 3' sequence of nucleotide pair(s) found at one or more polymorphic sites in a locus on a pair of homologous chromosomes in an individual. As used herein, genotype includes a full-genotype and/or a sub-genotype.

As used herein, the term "polynucleotide" shall mean any RNA or DNA, which may be unmodified or modified RNA or DNA. Polynucleotides include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, polynucleotide refers to triple-stranded regions comprising RNA or DNA or both RNA and DNA. The term polynucleotide also includes DNAs or RNAs containing one or more modified bases and DNAs or RNAs with backbones modified for stability or for other reasons.

As used herein the term "gene" shall mean a segment of DNA that contains all the information for the regulated biosynthesis of an RNA product, including promoters, exons, introns, and other untranslated regions that control expression.

As used herein the term "polypeptide" shall mean any polypeptide comprising two or more amino acids joined to each other by peptide bonds or modified peptide bonds, i.e., peptide isosteres. Polypeptide refers to both short chains, commonly referred to as peptides, glycopeptides or oligomers, and to longer chains, generally referred to as proteins. Polypeptides may contain amino acids other than the 20 gene-encoded amino acids. Polypeptides include amino acid sequences modified either by natural processes, such as post-translational processing, or by chemical modification techniques that are well known in the art. Such modifications are well described in basic texts and in more detailed monographs, as well as in a voluminous research literature.

As used herein, the term "polymorphic site" shall mean a position within a locus at which at least two alternative sequences are found in a population, the most frequent of which has a frequency of no more than 99%.

As used herein, the term "nucleotide pair" shall mean the nucleotides found at a polymorphic site on the two copies of a chromosome from an individual.

As used herein, the term "phased" means, when applied to a sequence of nucleotide pairs for two or more polymorphic sites in a locus, the combination of nucleotides present at those polymorphic sites on a single copy of the locus is known.

In order to deduce a correlation between clinical response to a treatment and a genotype or haplotype, it is necessary to obtain data on the clinical responses exhibited by a population of individuals who received the treatment, hereinafter the "clinical population". This clinical data may be obtained by analyzing the results of a clinical trial that has already been run and/or the clinical data may be obtained by designing and carrying out one or more new clinical trials.

As used herein, the term "clinical trial" means any research study designed to collect clinical data on responses to a particular treatment, and includes, but is not limited to, Phase I, II and III clinical trials. Standard methods are used to define the patient population and to enroll subjects.

As used herein the term "locus" shall mean a location on a chromosome or DNA molecule corresponding to a gene or a physical or phenotypic feature.

The therapeutic treatment of interest is administered to each individual in the trial population and each individual's response to the treatment is measured using one or more predetermined criteria. It is contemplated that in many cases, the trial population will exhibit a range of responses and that the investigator will choose the number of responder groups, e.g., low, medium and high, made up by the various responses. In addition, the gene for each individual in the trial population is genotyped and/or haplotyped, which may be done before or after administering the treatment.

*The Detection of Nucleic Acids and Proteins as Markers.* In a particular embodiment, the level of mRNA corresponding to the marker can be determined both by *in situ* and by *in vitro* formats in a biological sample using methods known in the art. The term "biological sample" is intended to include tissues, cells, biological fluids and isolates thereof, isolated from a subject, as well as tissues, cells and fluids present within a subject. Many expression detection methods use isolated RNA. For *in vitro* methods, any RNA isolation technique that does not select against the isolation of mRNA can be utilized for the purification of RNA from cells. See, *e.g*., Ausubel et al., Ed., Curr. Prot. Mol. Biol., John Wiley & Sons, NY (1987-1999). Additionally, large numbers of tissue samples can readily be processed using techniques well-known to those of skill in the art, such as, e.g., the single-step RNA isolation process of U.S. Pat. No. 4,843,155.

The isolated mRNA can be used in hybridization or amplification assays that include, but are not limited to, Southern or Northern analyses, PCR analyses and probe arrays. One preferred diagnostic method for the detection of mRNA levels involve contacting the isolated mRNA with a nucleic acid molecule (probe) that can hybridize to the mRNA encoded by the gene being detected. The nucleic acid probe can be, e.g., a full-length cDNA, or a portion thereof, such as an oligonucleotide of at least 7, 15, 30, 50, 100, 250 or 500 nucleotides in length and sufficient to specifically hybridize under stringent conditions to a mRNA or genomic DNA encoding a marker. Other suitable probes for use in the diagnostic assays of the invention are described herein. Hybridization of an mRNA with the probe indicates that the marker in question is being expressed.

In one format, the mRNA is immobilized on a solid surface and contacted with a probe, for example, by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the markers of the present disclosure.

An alternative method for determining the level of mRNA corresponding to a marker of the present disclosure in a sample involves the process of nucleic acid amplification, e.g., by RT-PCR (the experimental embodiment set forth in Mullis, U.S. Pat. No. 4,683,202 (1987); ligase chain reaction, Barany (1991), *supra;* self-sustained sequence replication, Guatelli et al., Proc. Natl. Acad. Sci. USA, Vol. 87, pp. 1874-1878 (1990); transcriptional amplification system, Kwoh et al., Proc. Natl. Acad. Sci. USA, Vol. 86, pp. 1173-1177 (1989); Q-Beta Replicase, Lizardi et al., Biol. Technology, Vol. 6, p. 1197 (1988); rolling circle replication, U.S. Pat. No. 5,854,033 (1988); or any other nucleic acid amplification method, followed by the detection of the amplified molecules using techniques well-known to those of skill in the art. These detection schemes are especially useful for the detection of the nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10-30 nucleotides in length and flank a region from about 50-200 nucleotides in length. Under appropriate conditions and with appropriate reagents, such primers permit the amplification of a nucleic acid molecule comprising the nucleotide sequence flanked by the primers.

For *in situ* methods, mRNA does not need to be isolated form the cells prior to detection. In such methods, a cell or tissue sample is prepared/processed using known histological methods. The sample is then immobilized on a support, typically a glass slide, and then contacted with a probe that can hybridize to mRNA that encodes the marker.

As an alternative to making determinations based on the absolute expression level of the marker, determinations may be based on the normalized expression level of the marker. Expression levels are normalized by correcting the absolute expression level of a marker by comparing its expression to the expression of a gene that is not a marker, e.g., a housekeeping gene that is constitutively expressed. Suitable genes for normalization include housekeeping genes, such as the actin gene or epithelial cell-specific genes. This normalization allows the comparison of the expression level in one sample, e.g., a patient sample, to another sample or between samples from different sources.

Alternatively, the expression level can be provided as a relative expression level. To determine a relative expression level of a marker, the level of expression of the marker is determined for 10 or more samples of normal versus disease biological samples, preferably 50 or more samples, prior to the determination of the expression level for the sample in question. The mean expression level of each of the genes assayed in the larger number of samples is determined and this is used as a baseline expression level for the marker. The expression level of the marker determined for the test sample (absolute level of expression) is then divided by the mean expression value obtained for that marker. This provides a relative expression level.

Preferably, the samples used in the baseline determination will be from patients who do not have the polymorphism. The choice of the cell source is dependent on the use of the relative expression level. Using expression found in normal tissues as a mean expression score aids in validating whether the marker assayed is specific (versus normal cells). In addition, as more data is accumulated, the mean expression value can be revised, providing improved relative expression values based on accumulated data.

*Detection of Polypeptides.* In another embodiment, a polypeptide corresponding to a marker is detected. A preferred agent for detecting a polypeptide is an antibody capable of binding to a polypeptide corresponding to a marker of the disclosure, preferably an antibody with a detectable label. Antibodies can be polyclonal, or more preferably, monoclonal. An intact antibody, or a fragment thereof, *e.g*., Fab or F(ab')₂ can be used. The term "labeled", with regard to the probe or antibody, is intended to encompass direct-labeling of the probe or antibody by coupling, i.e., physically linking, a detectable substance to the probe or antibody, as well as indirect-labeling of the probe or antibody by reactivity with another reagent that is directly-labeled. Examples of indirect labeling include detection of a primary antibody using a fluorescently-labeled secondary antibody and end-labeling of a DNA probe with biotin such that it can be detected with fluorescently-labeled streptavidin.

Proteins from individuals can be isolated using techniques that are well-known to those of skill in the art. The protein isolation methods employed can, e.g., be such as those described in Harlow & Lane (1988), *supra.*

A variety of formats can be employed to determine whether a sample contains a protein that binds to a given antibody. Examples of such formats include, but are not limited to, EIA; radioimmunoasay (RIA), Western blot analysis and ELISA. A skilled artisan can readily adapt known protein/antibody detection methods for use in determining whether cells express a marker of the present disclosure and the relative concentration of that specific polypeptide expression product in blood or other body tissues.

In one format, antibodies or antibody fragments, can be used in methods, such as Western blots or immunofluorescence techniques to detect the expressed proteins. In such uses, it is generally preferable to immobilize either the antibody or proteins on a solid support. Suitable solid phase supports or carriers include any support capable of binding an antigen or an antibody. Well-known supports or carriers include glass, polystyrene, polypropylene, polyethylene, dextran, nylon, amylases, natural and modified celluloses, polyacrylamides, gabbros and magnetite.

One skilled in the art will know many other suitable carriers for binding antibody or antigen, and will be able to adapt such support for use with the present invention. For example, protein isolated from patient cells can be run on a polyacrylamide gel electrophoresis and immobilized onto a solid phase support, such as nitrocellulose. The support can then be washed with suitable buffers followed by treatment with the detectably-labeled antibody. The solid phase support can then be washed with the buffer a second time to remove unbound antibody. The amount of bound label on the solid support can then be detected by conventional means and this measurement translated into a level or concentration of protein in blood or another body tissue.

The disclosure also encompasses kits for detecting the presence of a polypeptide or nucleic acid corresponding to a marker of the disclosure in a biological sample, e.g., any body fluid including, but not limited to, serum, plasma, lymph, cystic fluid, urine, stool, csf, acitic fluid or blood and including biopsy samples of body tissue. For example, the kit can comprise a labeled compound or agent capable of detecting a polypeptide or an mRNA encoding a polypeptide corresponding to a marker of the disclosure in a biological sample and means for determining the amount of the polypeptide or mRNA in the sample, *e.g*., an antibody which binds the polypeptide or an oligonucleotide probe which binds to DNA or mRNA encoding the polypeptide. Kits can also include instructions for interpreting the results obtained using the kit.

For antibody-based kits, the kit can comprise, *e.g*., 1) a first antibody, *e.g*., attached to a solid support, which binds to a polypeptide corresponding to a marker or the disclosure; and, optionally; and optionally 2) a second, different antibody which binds to either the polypeptide or the first antibody and is conjugated to a detectable label.

For oligonucleotide-based kits, the kit can comprise, *e.g*., 1) an oligonucleotide, *e.g*., a detectably-labeled oligonucleotide, which hybridizes to a nucleic acid sequence encoding a polypeptide corresponding to a marker of the disclosure, or 2) a pair of primers useful for amplifying a nucleic acid molecule corresponding to a marker of the disclosure.

The kit can also comprise, *e.g*., a buffering agent, a preservative or a protein-stabilizing agent. The kit can further comprise components necessary for detecting the detectable-label, *e.g*., an enzyme or a substrate. The kit can also contain a control sample or a series of control samples, which can be assayed and compared to the test sample. Each component of the kit can be enclosed within an individual container and all of the various containers can be within a single package, along with instructions for interpreting the results of the assays performed using the kit.

. *Kits.* The kits may contain a written product on or in the kit container. The written product describes how to use the reagents contained in the kit to predict whether a patient will respond effectively to macrolactam treatment, especially pimecrolimus treatment. In several embodiments, the use of the reagents can be according to the methods of the disclosure.

### EXAMPLE

### PHARMACOGENETIC ANALYSIS OF PATIENT RESPONSE TO THERAPY IN CLINICAL TRIAL: POTENTIAL ASSOCIATION OF PIMECROLIMUS EFFICACY WITH POLYMORPHISMS IN THE TNF GENE CLUSTER

*Genotyping selected candidate genes.* Forty-six patients, corresponding to 24 patients who received tacrolimus and 22 patients who received pimecrolimus, were analyzed for possible associations between genetic markers and treatment efficacy.

Thirty-one unique polymorphic sites in a total of 22 genes were analyzed. TABLE 1 lists the genotype frequencies for each SNP examined in the trial population that consented to participation in the pharmacogenetic analysis. Each SNP is identified by three pieces of information: (1) REF_ACC, or the Genbank accession number where genomic sequence for the gene can be found, (2) Position, which refers to the position of the nucleotide within the REF_ACC that was interrogated, and (3) TWT SNP#, which refers to the number assigned by Third Wave Technologies to the assay that was designed to assess the genotype at the position defined by items 1 and 2. Location refers to the site within the gene that harbors the polymorphism, if known.

**TABLE 1**

| List of polymorphisms examined in the pharmacogenomic analysis | | | | | | | |
|---|---|---|---|---|---|---|---|
| Gene Symbol | Gene Name | TWT SNP # | REF ACC | Allele 1 Freq. | Allele 2 Freq. | Position | Location |
| *ACE* | Angiotensin I converting enzyme | 103199 | AF1178569 | 0.50(T) | 0.50(C) | 10514 | |
| *ACE* | Angiotensin I converting enzyme | 103200 | AF1178569 | 0.56(A) | 0.44(G) | 14521 | |
| *CCR2* | chemokine (C-C motif) receptor 2 | 103240 | U80924 (SEQ ID NO:10) | 1.0(A) | 0.00(G) | 4201 | 3' UTR |
| *CCR2* | chemokine (C-C motif) receptor 2 | 47987 | U80924 (SEQ ID NO:10) | 0.77(G) | 0.23(A) | 46295 | Exon 1 |
| *CCR5+* | chemokine (C-C motif) receptor 5 | 128004 | U95626 | 1.00(T) | 0.00(A) | 61785 | Exon 2 |
| *CCR5* | chemokine (C-C motif) receptor 5 | 128005 | U95626 | 0.60(A) | 0.40(G) | 62035 | promoter |
| *CTLA4* | cytotoxic T-lymphocyte-ass ociated protein 4 | 128016 | M74363 | 0.56(A) | 0.44(G) | 1241 | Exon 1 |
| *ICAM1** | intercellular adhesion molecule 1 | 128063 | X59287 | 0.98(A) | 0.02(T) | 120 | Exon 2 |
| *ICAM1** | intercellular adhesion molecule 1 | 128062 | X59288 | 0.98(G) | 0.02(T) | 659 | Exon 4 |
| *IFNG* | Interferon gamma | 229376 | J00219 | 0.65(A) | 0.35(G) | 5644 | promoter |
| *IFNGR1*+ | Interferon gamma receptor 1 | 229419 | U19241 | 1.00(G) | 0.00(A) | 4020 | Exon 1 |
| *IFNGR2* | Interferon gamma receptor 2 | 252011 | AP000113 | 0.84(A) | 0.16(G) | 42786 | Exon 2 |
| *IL10* | Interleukin 10 | 229400 | X78437 | 0.67(A) | 0.33(G) | 8210 | 5'UTR |
| *IL3* | Interleukin 3 | 251975 | AF365976 | 0.61(C) | 0.39(T) | 1990 | Exon 1 |
| *IL3*+ | Interleukin 3 | 229368 | AF365976 | 1.00(T) | 0.00(C) | 3622 | |
| *IL4R* | Interleukin 4 receptor | 229406 | AF421857 | 0.52(C) | 0.48(T) | 13715 | Intron 5 |
| *IL5** | Interleukin 5 | 229372 | AF353265 | 0.98(G) | .02(A) | 2718 | |
| *IL8* | Interleukin 8 | 229405 | AF385628 | 0.55(C) | 0.45(T) | 4501 | Intron 2 |
| *ITGB2*+ | Integrin, beta 2 (LFA1) | 128081 | X64075 | 1.00(G) | 0.00(A) | 64 | Exon 5 |
| *LTA* | Lymphotoxin alpha | 229383 | M55913 (SEQ ID NO:11) | 0.59(C) | 0.41(A) | 800 | Exon 3 |
| *LTB** | Lymphotoxin beta | 128095 | L11016 (SEQ ID NO:12) | 0.91(C) | 0.09(A) | 5452 | |
| *MICA* | MHC class I polypeptide-related sequence A | 229390 | AF336081 | 0.98(A) | 0.02(G) | 106 | Exon 2 |
| *MICA* | MHC class I polypeptide-related sequence A | 251974 | AF336081 | 0.78(A) | 0.22(G) | 1612 | Exon 5 |
| *MICA* | MHC class I polypeptide-related sequence A | 229395 | AF336081 | 0.76(G) | 0.24(A) | 658 | Exon 3 |
| *NFATC2* | nuclear factor of activated T-cells, cytoplasmic, calcineurin-depen dent 2 | 252013 | AL035682 | 0.49(A) | 0.51(G) | 58458 | |
| *PLGC1* | Phospholipase C γ-1 | 229389 | AL022394 | 0.69(C) | 0.31(T) | 64001 | |
| *TGFB1* | Transforming growth factor β-1 | 128146 | X05839 | 0.73(C) | 0.27(T) | 629 | promoter |
| *TNF* | Tumor necrosis factor α | 128152 | M16441 (SEQ ID NO:7) | 0.79(T) | 0.21(C) | 3064 | promoter |
| *TNF* | Tumor necrosis factor α | 128150 | M16441 (SEQ ID NO:7) | 0.85(C) | 0.15(T) | 3238 | promoter |
| *TNF* | Tumor necrosis factor α | 128148 | M16441 (SEQ ID NO:7) | 0.80(G) | 0.20(A) | 3787 | promoter |
| *TNFSF6* | tumor necrosis factor receptor superfamily, member 6 | 128153 | X81335 | 0.58(G) | 0.42(A) | 1110 | promoter |

| | | | | | | | |
|---|---|---|---|---|---|---|---|
| + This SNP is not polymorphic in this patient population. * This population is not in Hardy Weinberg equilibrium for this SNP. | | | | | | | |

The *FKBP1A* gene (SEQ ID NO:13), which codes for FK506 Binding Protein 1A (macrophilin-12), the target of pimecrolimus and tacrolimus (FK506), was not genotyped, because the gene was found to contain no SNPs. No polymorphic sites in *FKBP1A* have been reported in public databases. We sequenced the *FKBP1A* gene to look for unknown SNPs, but found none.

To interrogate the genotype at each locus of interest, probe sets flanking the SNP were designed using publicly available databases such as OMIM, the SNP Consortium, LocusLink and dbSNP. The probe sets were synthesized by Third Wave Technologies, Inc. (TWT, Madison WI). Genotyping was performed with 60 ng of genomic DNA using the Invader® assay developed by Third Wave Technologies according to the manufacturer's instructions. Lyamichev V et al., Nat Biotechnol 17: 292-6 (1999) and Ryan D, Mol Diagn 4: 135-44 (1999).

*Genotyping of ASN60THR LTA.* To confirm the results of the Third Wave Technologies genotyping assay for ASN60THR *LTA* (TWT #229383), a nested polymerase chain reaction (PCR) approach was used. All oligonucleotides were purchased from Research Genetics (Huntsville, AL). First, a 481 bp fragment was amplified using primers with the following sequences: forward (5'-acaccacctgaacgtctcttc-3'; SEQ ID NO:14) reverse (5'-tctcaatccctgaggaagtgg-3'; SEQ ID NO:15). See Genbank accession number M55913 for the complete *LTA* sequence (SEQ ID NO:11). The amplicon was purified using Microcon 100 columns (Amlicon, Beverly, MA) and used as a template to amplify a 163 bp fragment using primers with the following sequences: forward (5'-tcagccaaaccttgagccctagag-3'; SEQ ID NO:16) and reverse (5'-atgtttaccaatgaggtgagcagcaggtttgcgg-3'; SEQ ID NO:17). Both PCR reactions were carried out in a Perkin Elmer 9700 thermocycler using 80 ng of genomic DNA, 60 ng of each primer, 1.0 Unit (U) of AmpliTaq polymerase (Perkin Elmer), and 200 µM dNTP (Pharmacia, Piscataway, N.J.) in a buffer consisting of 50 mM KCl, 10mM TRIS-HCl (pH 8.3), 1.5 mM MgCl and 0.01 % gelatin. The PCR cycles for both amplifications consisted of the following: 94°C, 5 min; 94°C, 30 sec, 57°C, 30 sec, and 72°C, 30 sec for 35 cycles; 72°C, 10 minutes, then 4°C. The 163 bp amplicon was digested with Fau I (New England Biolabs, Beverly MA), and electrophoresed on a 4% agarose gel. A G allele produced an uncut 163 bp fragment, while an A produced 35 and 128 bp fragments. Both the 128 and 163 bp fragments were seen in heterozygote individuals. All but two of the genotypes determined by Third Wave Technologies technology could be confirmed by restriction digest.

*Genotyping of (-1031) TNF.* To confirm the results of the genotyping assay for *TNF* -1031, a 1113 bp fragment of the *TNF* promoter was PCR amplified and sequenced for each sample. PCR primers had the following sequences: forward (5'-TGGGAGTGAGAACTTCCCAG-3'; SEQ ID NO:18) and reverse (5'-TGAGCTCATCTGGAGGAAGC-3'; SEQ ID NO:19). See Genbank accession number M16441 for the complete sequence of human *TNF.* PCR reactions were carried out using the same conditions described above. PCR products were purified through Microcon 100 (Amlicon, Beverly, MA) columns.

Following purification of the amplicon, 5 ng per 100 bp were used for sequencing with the ABI Prism Dye Terminator Cycle Sequencing kit as recommended (Perkin Elmer, Foster City, CA). Sequencing reactions contained either forward (5'-TGGGAGTGAGAACTTCCCAG-3'; SEQ ID NO:20) or reverse (5'-CTTAAACGTCCCCTGTATTC-3'; SEQ ID NO:21) primer and were run as follows: 96°C, 5 min; 96°C, 10 sec, 50°C, 5 sec, and 60°C, 4 min for 25 cycles; then 4°C. Sequencing reactions were purified with Centrisep spin columns (Princeton Separation, Adelphia, NJ) and run on the ABI 373A sequencer. DNA sequences were analyzed using ABI Prism Sequence Analysis V3.3 software.

*Statistical analysis.* Investigator global assessment (IGA) scores were used as the primary marker of efficacy in the pharmacogenetic studies. IGA scores at the end of the Investigator Blinded phase of the trial (Day 43) were used as the main time-point for comparison. Last observation carried forward imputations were only required for one subject at day 43. In this case, there was no IGA score available after Day 29, and the score on this day was used in place of a day 43 IGA score. IGA scores were re-coded, as was done to assess efficacy in the trial itself. An IGA score of 0 or 1 was considered successful treatment and IGA scores of 2-5 were counted as treatment failure. The severity of pruritus was also used as a marker of efficacy. Pruritus scores were dichotomized into absence (score = 0) or presence (score = 1, 2 or 3) of pruritus. The fisher's exact model was employed to examine the genotype effect on each efficacy variable. All statistical analyses were done using SAS version 8.2 software.

To correct for multiple testing, the Bonferroni correction method was applied to all results. The equation used to correct significance scores (p values) is: Bonferroni = *P X* η, where *P* = p value for association between genotype and efficacy of treatment and η = number of polymorphic markers genotyped in the trial.

*Demographics trial participants.* As demonstrated in TABLE 2, the trial subjects that consented to the pharmacogenomics (PG) analysis were representative of the patient population in the trial overall in terms of gender, age, ethnicity, and response to treatment.

**TABLE 2**

| Demographics of pharmacogenomic analysis participants compared to trial subjects | | | | | |
|---|---|---|---|---|---|
| | | | | Pharmacogenomics Samples* | |
| | | Trial⁺ tacrolimus | pimecrolimus | tacrolimus | pimecrolimus |
| Age (years) Race | | 7.8 | 8.1 | 8.7 | 8.5 |
| | Caucasian | 31(41%) | 45 (63%) | 12 (48%) | 17 (77%) |
| | Black | 14 (20%) | 13 (18%) | 7 (28%) | 4 (18%) |
| | Oriental | 4 (6%) | 3 (4%) | 1 (4%) | 0 |
| | Other | 21 (30%) | 10 (14%) | 5 (20%) | 1 (5%) |
| Gender | | | | | |
| | male | 31 (44%) | 31 (44%) | 12 (48%) | 11 (50%) |
| | female | 39 (56%) | 40 (56%) | 14 (52%) | 11 (50%) |
| IGA (baseline)^{$} | | 3: 69 | 3: 70 | 3: 24 | 3: 21 |
| | | 2: 1 | 2: 1 | | 2: 1 |
| Efficacy (IGA)^{#} | | 27 (40%) | 22 (32%) | 13 (52%) | 8 (36%) |
| Pruritus (day 1) | | 0: 1 (1%) | 0: 2 (3%) | | |
| | | 1: 13 (19%) | 1: 14 (20%) | 1: 6 (24%) | 1: 4 (18%) |
| | | 2: 33 (47%) | 2: 25 (35%) | 2: 11 (44%) | 2: 9 (41%) |
| | | 3: 23 (33%) | 3: 30 (42%) | 3: 8 (31%) | 3: 9 (41%) |
| Pruritus (day 43) | | 0: 11 (16%) | 0: 9 (13%) | 0: 5 (20%) | 0: 2 (10%) |
| | | 1: 35 (50%) | 1: 35 (49%) | 1: 10 (40%) | 1: 14 (67%) |
| | | 2: 19 (27%) | 2: 15 (21%) | 2: 7 (28%) | 2: 3 (14%) |
| | | 3: 3 (4%) | 3: 10 (14%) | 3: 3 (12%) | 3: 2 (10%) |

| | | | | | |
|---|---|---|---|---|---|
| + There were 70 subjects in the tacrolimus arm of the trial and 71 subjects in the pimecrolimus arm of the trial * 24 subjects that took tacrolimus and 22 patients that took pimecrolimus were analyzed in the pharmacogenomic analysis. $ IGA scores were taken at screening. # Number and (percent) of patients that experienced efficacy, defined as an IGA score of 0 or 1 recorded on day 43 of treatment (end of blinded portion of the trial). | | | | | |

*Association between (-1031) TNF and macrolactam efficacy.* As stated above, the primary efficacy variable used in statistical analysis of each genetic marker was a re-coded IGA score. A total of 31 loci in 22 genes were genotyped. Of the 31 loci that were genotyped, 5 were not polymorphic in the pharmacogenonic analysis population and were dropped from the analysis. See, TABLE 1, above. Thus, the penalty for multiple testing in this analysis is 26.

When the trial was analyzed as a whole (both arms combined), 1 of the 26 SNPs analyzed showed a statistically significant association with efficacy. Tumor necrosis factor alpha, *TNF,* is located in the gene-rich MHC cluster on 6p21.3 *(see,* FIG. 1), and many of the genes in this region play important roles in inflammatory processes. The promoter of *TNF* is highly polymorphic. A SNP located -1031 bp from the transcription start site of the gene associated with efficacy (p=0.04, see TABLE 3, below). These data demonstrate that patients that harbor a C allele (either CC homozygotes or CT heterozygotes) at (-1031) *TNF* were less likely to respond to treatment (4/17; 24% success rate) than were TT patients (17/29; 59% success rate).

**TABLE 3**

| Association between (-1031) *TNF* and macrolactam efficacy | | | | |
|---|---|---|---|---|
| | (-1031) *TNF* Genotype | | | |
| Response to treatment | CC | CT | TT | Total |
| No Success (IGA ≥ 2) | 2 (*67%*) | 11 (*79%*) | 12 (*41%*) | 25 |
| Success (IGA = 0 or 1) | 1 (*33%*) | 3 (*21%*) | 17 (*59%*) | 21 |
| Total | 3 | 14 | 29 | 46 |

TABLE 3 demonstrates the number of subjects with each genotype that did or did not respond to treatment (referred to as "success" or "no success", respectively), as determined by IGA scores on day 43 of treatment. Each square contains the number of individuals with the given genotype that fit the response category, followed by the *percent.* P value for the association is 0.04 (Fisher's Exact).

We next examined the association between (-1031) *TNF* and efficacy for tacrolimus and pimecrolimus treatment independently. As seen in TABLE 4, those subjects who responded to pimecrolimus can be segregated from non-responders based on (-1031) *TNF.* Only subjects with a TT genotype (SEQ ID NO:1) at (-1031) *TNF* responded to pimecrolimus treatment. None of the ten subjects with a C allele (SEQ ID. NO:2; CC or CT) responded to pimecrolimus treatment. While response rate among CC and CT patients was 0%, 67% of the TTs experienced remission of their atopic dermatitis after taking pimecrolimus.

**TABLE 4**

| Association between (-1031) *TNF* and pimecrolimus efficacy | | | | |
|---|---|---|---|---|
| | (-1031) *TNF* Genotype | | | |
| Response to pimecrolimus | CC | CT | TT | Total |
| No success (IGA ≥ 2) | 1 (*100%*) | 9 (*100%*) | 4 (*33%*) | 14 |
| Success (IGA = 0 or 1) | 0 (*0%*) | 0 (*100%*) | 8 (*67%*) | 8 |
| Total | 1 | 9 | 12 | 22 |

TABLE 4 demonstrates the number of pimecrolimus treated subjects with each genotype that did or did not respond to treatment (referred to as "success" or "no success", respectively), as determined by IGA scores on day 43 of treatment. Each square contains the number of individuals with the given genotype that fit the response category, followed by the *percent.* P value for the association is 0.003 (Fisher's Exact).

However, genotype at this locus does not appear to influence tacrolimus efficacy. See, TABLE 5, below. Indeed, of the subjects that used tacrolimus, responders appear to be just as likely to be TT as CT or CC. Thus, the association seen between efficacy in the trial as a whole and (-1031) *TNF* holds only for pimecrolimus and not macrolactams in general.

**TABLE 5**

| Association between (-1031) *TNF* and tacrolimus efficacy | | | | |
|---|---|---|---|---|
| | (-1031) *TNF* Genotype | | | |
| Response to tacrolimus | CC | CT | TT | Total |
| No success (IGA ≥ 2) | 1 (*50%*) | 2 (*40%*) | 8 (*47%*) | 11 |
| Success (IGA = 0 or 1) | 1 (*50%*) | 3 (*60%*) | 9 (53%) | 13 |
| Total | 2 | 5 | 17 | 24 |

TABLE 5 demonstrates the number of tacrolimus treated subjects with each genotype that did or did not respond to treatment (referred to as "success" or "no success", respectively), as determined by IGA scores on day 43 of treatment. Each square contains the number of individuals with the given genotype that fit the response category, followed by the *percent.* There was no association found between (-1031) *TNF* genotype response to tacrolimus; the P value for the association is 1.0 (Fisher's Exact).

*Association between ASN60THR LTA and macrolactam efficacy.* When both arms of the trial were analyzed together, an association with efficacy was only found with (-1031) *TNF.* Interestingly, the data for a second polymorphism suggested a trend toward significance. A C→A polymorphism in exon 3 of *LTA* (SEQ ID NO:3), which results in the production of threonine instead of asparagine at the 60^{th} amino acid of the protein (ASN60THR), was associated with efficacy in the clinical trial (p=0.07, see TABLE 6, below). *LTA* is tandemly located with *TNF* on 6p21.3, and numerous reports suggest that markers in *TNF* and *LTA* are in strong linkage disequilibrium. Bouma G et al., Scand J Immunol 43: 456-63 (1996); Noguchi E et al., Am J Respir Crit Care Med 166: 43-6 (2002); .Moffatt M & Cookson W. Hum Molec Genet 6: 551-4 (1997); Messer G et al., J Exp Med 173: 209-19 (1991). Genotypes at this locus were determined in the pharmacogenomic analysis participants using an Invader Assay (TWT#229383) and confirmed by restriction digest. The data demonstrate that patients harboring an A, the wild-type allele(SEQ ID NO:3), at this locus were more likely to respond to treatment; the response rate among subjects with an A allele (SEQ ID NO:4); AA or AC) was 57% (17/30), while the only 25% (4/16) of those subjects with a CC genotype experienced efficacy in the trial.

**TABLE 6**

| Association between ASN60THR LTA and macrolactam efficacy (both arms combined) | | | | |
|---|---|---|---|---|
| | ASN60THR *LTA* Genotype | | | |
| Response to Treatment | AA | AC | CC | Total |
| No Success (IGA ≥ 2) | 3 (*30%*) | 10 (*50%*) | 12 (*75%*) | 25 |
| Success (IGA = 0 or 1) | 7 (*70%*) | 10 (*50%*) | 4 (*25%*) | 21 |
| Total | 10 | 20 | 16 | 46 |

TABLE 6 demonstrates the number of subjects in the clinical trial with each genotype that did or did not respond to treatment (referred to as "success" or "no success", respectively), as determined by IGA scores on day 43 of treatment. Each square contains the number of individuals with the given genotype that fit the response category, followed by the *percent.* While a significant association was not found (P value = 0.07, Fisher's Exact), the P value approaches significance and is suggestive of a possible association.

We next examined the association between ASN60THR *LTA* and each arm of the clinical trial independently. As anticipated, a significant association was found for pimecrolimus (P=0.02, see TABLE 7, below), and a dosage effect was seen with the polymorphism. Patients with a CC genotype at this locus responded poorly to pimecrolimus (1/8 or 11% were successfully treated). However, patients with an AC genotype responded better (4/10 or 40% response rate), and all patients with an AA genotype responded to the treatment (3/3).

**TABLE 7**

| Association between ASN60THR *LTA* and pimecrolimus efficacy | | | | |
|---|---|---|---|---|
| | ASN60THR *LTA* Genotype | | | |
| Response to pimecrolimus | AA | AC | CC | Total |
| No success (IGA ≥ 2) | 0 (*0%*) | 6 (*60%*) | 8 (*89%*) | 13 |
| Success (IGA = 0 or 1) | 3 (*100%*) | 4 (*40%*) | 1 (*11%*) | 7 |
| Total | 3 | 10 | 9 | 22 |

TABLE 7 demonstrates the number of pimecrolimus treated subjects with each genotype that did or did not respond to treatment (referred to as "success" or "no success", respectively), as determined by IGA scores on day 43 of treatment. Each square contains the number of individuals with the given genotype that fit the response category, followed by the *percent.* The P value for the association is 0.02 (Fisher's Exact).

A similar relationship was not found between ASN60THR *LTA* and tacrolimus efficacy. As seen in TABLE 8, the presence of the A allele at ASN60THR *LTA* had no influence on response to tacrolimus. As was found with (-1031) *TNF,* the association between ASN60THR *LTA* and efficacy in the clinical trial is driven by the pimecrolimus arm of the trial.

**TABLE 8**

| Association between ASN60THR *LTA* and tacrolimus efficacy | | | | |
|---|---|---|---|---|
| | ASN60THR *LTA* Genotype | | | |
| Response to tacrolimus | AA | AC | CC | Total |
| No success (IGA ≥ 2) | 3 (*43%*) | 4 (*40%*) | 4 (*57%*) | 11 |
| Success (IGA = 0 or 1) | 4 (*57%*) | 6 (*60%*) | 3 (*42%*) | 13 |
| Total | 7 | 10 | 7 | 24 |

TABLE 8 demonstrates the number of tacrolimus treated subjects with each genotype that did or did not respond to treatment (referred to as "success" or "no success", respectively), as determined by IGA scores on day 43 of treatment. Each square contains the number of individuals with the given genotype that fit the response category, followed by the *percent.* There was no association found between ASN60THR *LTA* genotype and response to tacrolimus; the P value for the association is 1.0 (Fisher's Exact).

*Association between polymorphic markers and relief from pruritus.* A secondary efficacy variable used in the trial was pruritus. An analysis was done to see if ASN60THR *LTA* and (-1031) *TNF* associated with this efficacy variable (assessed on day 43). Results of the analysis for ASN60THR *LTA* and (-1031) *TNF* are shown in TABLES 9 and 10, respectively.

Few subjects (7/46) in the pharmacogenomics group experienced relief from itching. Findings for the primary efficacy variable (IGA) suggested that the presence of a C allele at ASN60THR *LTA* decreased the likelihood of response to treatment. While a statistically significant association between ASN60THR *LTA* and pruritus was not found, no subject that was homozygous CC at this locus experienced relief from itching as a result of treatment in this trial. For (-1031) *TNF*; while analysis of the primary efficacy variable IGA suggested that TT subjects were far more likely to respond to treatment, the same cannot be said when pruritus scores are analyzed for the trial as a whole. The two arms of the trial could not be separated for analysis of pruritus because the number of responders is too small. Only two pimecrolimus subjects in the pharmacogenomic analysis samples experienced relief from itching. However, both of the pimecrolimus subjects that experienced efficacy with respect to pruritus were TT at (-1031) *TNF.*

**TABLE 9**

| Association between ASN60THR *LTA* and pruritus (both arms combined). | | | | |
|---|---|---|---|---|
| | ASN60THR *LTA* Genotype | | | |
| Response to treatment | AA | AC | CC | Total |
| No Success (Pru=1,2,3) | 7 (*70%*) | 16 (*80%*) | 16 (*100%*) | 39 |
| Success (Pru=0) | 3 (*30%*) | 4 (*20%*) | 0 (*0%*) | 7 |
| Total | 10 | 16 | 16 | 46 |

TABLE 9 demonstrates the number of subjects with each genotype at the ASN60THR *LTA* locus studied that did or did not respond to treatment, as determined by pruritus scores on day 43 of treatment. Treatment was considered successful if the patient reported no pruritus (score = 0). Each square contains the number of individuals with the given genotype that fit the response category, followed by the *percent.* A significant association was not found between ASN60THR *LTA* genotype and relief from pruritus (P=0.06, Fisher's Exact), however the P value for the association approached significance.

**TABLE 10**

| Association between (-1031) *TNF* and pruritus following treatment (both arms combined) | | | | |
|---|---|---|---|---|
| | (-1031) *TNF* Genotype | | | |
| Response to treatment | CC | CT | TT | Total |
| No success (Pru=1,2,3) | 3 (*100%*) | 12 (*86%*) | 24 (*83%*) | 39 |
| Success (Pru = 0) | 0 (*0%*) | 2 (*14%*) | 5 (*17%*) | 7 |
| Total | 3 | 14 | 29 | 46 |

TABLE 10 demonstrates the number of subjects with each genotype at (-1031) *TNF* that did or did not respond to treatment, as determined by pruritus scores on day 43 of treatment. Treatment was considered successful if the patient reported no pruritus (score = 0). Each square contains the number of individuals with the given genotype that fit the response category, followed by the *percent.* No association was found (P=1.0, Fisher's Exact).

*Genotypes at (-1031) TNF and ASN60THR LTA.* Genotypes of anonymized patients at these two 6p21.3 loci were obtained. Only subjects with a TT genotype at (-1031) TNF responded to pimecrolimus therapy. The observation that individuals that are TT homozygotes for (-1031) TNF are either AA homozygotes, AC heterozygotes, or CC homozygotes or for ASN60THR LTA show that (-1031) TNF and ASN60THR are not in complete linkage disequilibrium.

**TABLE 11**

| Nucleotide sequence surrounding the ASN60THR LTA and (-1031) TNF polymorphisms | | | |
|---|---|---|---|
| Gene | Allele 1 | Allele 2 | Surrounding Sequence |
| *LTA* | C | A | |
| *TNF* | T | C | |

*Associations between pimecrolimus and tacrolimus and other markers typed.* All of the other molecular markers typed were checked to see if they segregated responders from non-responders for each arm of the trial independently. No additional associations were found for pimecrolimus, though one association was found for tacrolimus. A G→A polymorphism in the coding region of *CCR2* (SEQ ID NO:6; see TWT #47987), which results in a change in a valine to isoleucine (VAL64ILE), associated with response to treatment with tacrolimus (p=0.04, see TABLE 12,). The P value for the association between pimecrolimus efficacy and VAL64ILE *CCR2* was 1.0 (Fisher's Exact).

*CCR2* encodes chemokine (C-C) motif receptor 2, a receptor for monocyte chemoattractant protein-1 and a chemokine that mediates monocyte infiltration in inflammatory diseases. *CCR2* encodes for both known isoforms of the receptor for monocyte chemoattractant protein-1 (MCP-1, also known as SCYA2), a chemokine which specifically mediates monocyte chemotaxis. MCP-1 has been shown to be involved with inflammatory diseases like rheumatoid arthritis and atherosclerosis. Boring L et al., Nature 394:894-7 (1998). The VAL64ILE polymorphism occurs in the first transmembrane region of CCR2 and has been studied in the context of HIV-1 infection and AIDS. Mummidi S et al., Nature Med 4: 786-93 (1998). The association found was p=0.04.

These data demonstrate that an A allele at VAL64ILE *CCR2* were more likely to respond to tacrolimus treatment; 8/10 or 80% of AG subjects experienced efficacy, as compared to 4/13 or 31% of GG subjects. (There were no AA subjects in the population). This association is weaker than the associations seen with pimecrolimus efficacy, particularly for (-1031) *TNF* and pimecrolimus efficacy (p=0.003).

**TABLE 12**

| Association between VAL63ILE *CCR2* (TWT# 47987) and tacrolimus efficacy | | | | |
|---|---|---|---|---|
| VAL63ILE *CCR2* Genotype | | | | |
| Response to tacrolimus | AA | AG | GG | Total |
| No success (IGA ≥ 2) | 0 | 2 (*20%*) | 9 (*69%*) | 11 |
| Success (IGA = 0 or 1) | 0 | 8 (*80%*) | 4 (*31%*) | 12 |
| Total | 0 | 10 | 13 | 23 |

TABLE 12 demonstrates the number of tacrolimus treated subjects with each genotype at VAL63ILE *CCR2* that did or did not respond to treatment (determined by IGA scores on day 43 of treatment). Each square contains the number of individuals with the given genotype that fit the response category, followed by the *percent.* P value for the association was 0.04 (Fisher's Exact).

*LTB* (SEQ ID NO 12; see TWT # 125095) is also located in the *TNF* gene cluster on 6p21.3. A polymorphism in *LTB* was also typed in this analysis (SEQ ID NO:22). As shown below, however, there is no association between TWT#125095 and efficacy in the clinical trial. No association was found when pimecrolimus and tacrolimus subjects were analyzed independently, either.

**TABLE 13**

| Relationship between efficacy in trial (both arms combined) and *LTB* (TWT#128095); data demonstrate no association (p=1.0, Fisher's Exact) | | | |
|---|---|---|---|
| *LTB* (TWT#128095) | | | |
| Response to treatment | AC | CC | Total |
| No success (IGA ≥ 2) | 3 (*50%*) | 20 (*54%*) | 23 |
| Success (IGA = 0 or 1) | 3 (*50%*) | 17 (*46%*) | 20 |
| Total | 16 | 26 | 43 |

**TABLE 14**

| Relationship between efficacy in among pimecrolimus-treated subjects and *LTB* (TWT#128095); data demonstrate no association (p=1.0, Fisher's Exact) | | | |
|---|---|---|---|
| *LTB* (TWT#128095) | | | |
| Response to pimecrolimus | AC | CC | Total |
| No success (IGA ≥ 2) | 0 (*50%*) | 12 (*63%*) | 12 |
| Success (IGA = 0 or 1) | 1 *(100%)* | 7 (*37%*) | 8 |
| Total | 1 | 19 | 20 |

**TABLE 15**

| Relationship between efficacy in among tacrolimus-treated subjects and *LTB* (TWT#128095); data demonstrate no association (p=1.0, Fisher's Exact) | | | |
|---|---|---|---|
| *LTB* (TWT#128095) | | | |
| Response to tacrolimus | AC | CC | Total |
| No success (IGA ≥ 2) | 3 (*60%*) | 8 (*44%*) | 11 |
| Success (IGA = 0 or 1) | 2 (*40%*) | 10 (*56%*) | 12 |
| Total | 5 | 18 | 23 |

These data help to localize the genetic region that is responsible for response to pimecrolimus; the biologically relevant polymorphism does not appear to be localized proximal to *TNF.*

In summary, either *LTA* or *TNF* plays an important role in determining efficacy of pimecrolimus in the treatment of pediatric atopic dermatitis. By contrast, response to tacrolimus appears to be influenced by other biological pathways. Thus, different biological mechanisms influence response to pimecrolimus and tacrolimus. While pimecrolimus and tacrolimus have substantial structural similarities and both target macrophilin-12 with the ultimate effect of inhibiting calcineurin, the two compounds are known to have distinct properties. Nghiem P et al., J Am Acad Dematol 46: 228-241 (2002) and references cited therein.

## Claims

1. Use of pimecrolimus in the manufacture of a medicament for the treatment of pediatric atopic dermatitis in a selected patient population, wherein the patient population is selected among those having either
(i) a TT genotype at a locus located -1031 from the transcription start site of the human *TNF* gene, or
(ii) having a AA or AC genotype at the ASN60THR *LTA* locus of the human *LTA* gene.

2. Use of tacrolimus in the manufacture of a medicament for the treatment of pediatric atopic dermatitis in a selected patient population, wherein the patient population is selected among those having the AG genotype at the VAL64ILE *CCR2* locus of the human *CCR2* gene.

## Patentansprüche

1. Verwendung von Pimecrolimus bei der Erstellung eines Medikaments zur Behandlung von atopischer Dermatitis beim Kind in einer ausgewählten Patientenpopulation, wobei die Patientenpopulation ausgewählt ist aus denen, die entweder
(i) ein TT-Genotyp an einer -1031 von der Transkriptionsstartstelle des humanen *TNF*-Gens befindlichen Stelle oder
(ii) einen AA- oder AC-Genotyp an der ASM60THR-*LTA*-Stelle des humanen *LTA*-Gens
aufweisen.

2. Verwendung von Tacrolimus bei der Herstellung eines Medikaments zur Behandlung von atopischer Dermatitis beim Kind in einer ausgewählten Patientenpopulation, wobei die Patientenpopulation ausgewählt ist aus denen, die einen AG-Genotyp an der VAL64ILE-*CCR2*-Stelle des humanen *CCR2*-Gens aufweisen.

## Revendications

1. Utilisation de pimécrolimus dans la fabrication d'un médicament destiné au traitement de la dermatite atopique pédiatrique chez une population de patients choisie, **caractérisée en ce que** la population de patients est choisie parmi celles ayant soit
(i) un génotype TT à un locus situé à -1031 du site d'initiation de la transcription du gène *TNF* humain, soit
(ii) ayant un génotype AA ou AC an locus ASN60THR *LTA* gène *LTA* humain.

2. Utilisation de tacrolimus dans la fabrication d'un médicament destiné au traitement de la dermatite atopique pédiatrique chez une population de patients choisie, **caractérisée en ce que** la population de patients est choisie parmi celle ayant le génotype AG au locus VAL64ILE *CCR2* du gène *CCR2* humain.


### SEQUENCE LISTING

<110> Novartis AG
   McCullough, Karen
   Ide, Susan
   Lavedan, Christian
<120> USE OF GENETIC POLYMORPHISMS THAT
   ASSOCIATE WITH EFFICACY OF TREATMENT OF INFLAMMATORY DISEASE
<130> DV/4-33389A
<150> 60/508,971 <151> 2003-10-06
<160> 22
<170> FastSEQ for windows version 4.0
<210> 1
   <211> 53
   <212> DNA
   <213> Homo sapiens
<220>
   <221> variation
   <222> (1)...(53)
   <223> TNF locus variant (T at position -1031)
   <221> variation
   <222> (23)...(0)
   <223> T
<400> 1
   agcaaaggag aagctgagaa gatgaaggaa aagtcagggt ctggaggggc ggg 53
<210> 2
   <211> 53
   <212> DNA
   <213> Homo sapiens
<220>
   <221> variation
   <222> (1)...(53)
   <223> TNF locus variant (C at position -1031)
   <221> variation
   <222> (23)...(0)
   <223> C
<400> 2
   agcaaaggag aagctgagaa gacgaaggaa aagtcagggt ctggaggggc ggg 53
<210> 3
   <211> 48
   <212> DNA
   <213> Homo sapiens
<220>
   <221> variation
   <222> (1)...(48)
   <223> LTA locus variant (C)
   <221> variation
   <222> (20)...(0)
   <223> C
<400> 3
   gtgagcagca ggtttgaggc tgctgtgggc aagatgcatc ttggggtg 48
<210> 4
   <211> 48
   <212> DNA
   <213> Homo sapiens
<220>
   <221> variation
   <222> (1)...(48)
   <223> LTA locus variant (A; ASN60THR)
   <221> variation
   <222> (20)...(0)
   <223> A
<400> 4
   gtgagcagca ggtttgagga tgctgtgggc aagatgcatc ttggggtg 48
<210> 5
   <211> 50
   <212> DNA
   <213> Homo sapiens
<220>
   <221> variation
   <222> (1)...(50)
   <223> CCR2 locus variant (G)
   <221> variation
   <222> (10)...(0)
   <223> G
<400> 5
   atgctggtcg tcctcatctt aataaactgc aaaaagctga agtgcttgac 50
<210> 6
   <211> 50
   <212> DNA
   <213> Homo sapiens
<220>
   <221> variation
   <222> (1)...(50)
   <223> CCR2 locus variant (A; VAL64ILE)
   <221> variation
   <222> (10)...(0)
   <223> A
<400> 6
   atgctggtca tcctcatctt aataaactgc aaaaagctga agtgcttgac 50
<210> 7
   <211> 702
   <212> DNA
   <213> Homo sapiens
<220>
   <221> CDS
   <222> (1)...(702)
   <223> Tumor necrosis factor alpha (TNFalpha) mRNA coding region
<400> 7
   atg agc act gaa agc atg atc cgg gac gtg gag ctg gcc gag gag gcg 48
   Met Ser Thr Glu Ser Met Ile Arg Asp Val Glu Leu Ala Glu Glu Ala
   1               5                   10                  15
   ctc ccc aag aag aca ggg ggg ccc cag ggc tcc agg cgg tgc ttg ttc      96
   Leu Pro Lys Lys Thr Gly Gly Pro Gln Gly Ser Arg Arg Cys Leu Phe
               20                  25                  30
   ctc agc ctc ttc tcc ttc ctg atc gtg gca ggc gcc acc acg ctc ttc     144
   Leu Ser Leu Phe Ser Phe Leu Ile Val Ala Gly Ala Thr Thr Leu Phe
           35                  40                  45
   tgc ctg ctg cac ttt gga gtg atc ggc ccc cag agg gaa gag ttc ccc     192
   Cys Leu Leu His Phe Gly Val Ile Gly Pro Gln Arg Glu Glu Phe Pro
       50                  55                  60
   agg gac ctc tct cta atc agc cct ctg gcc cag gca gtc aga tca tct     240
   Arg Asp Leu Ser Leu Ile Ser Pro Leu Ala Gln Ala Val Arg Ser Ser
   65                  70                  75                  80
   tct cga acc ccg agt gac aag cct gta gcc cat gtt gta gca aac cct     288
   Ser Arg Thr Pro Ser Asp Lys Pro Val Ala His Val Val Ala Asn Pro
                   85                  90                  95
   caa gct gag ggg cag ctc cag tgg ctg aac cgc cgg gcc aat gcc ctc     336
   Gln Ala Glu Gly Gln Leu Gln Trp Leu Asn Arg Arg Ala Asn Ala Leu
               100                 105                 110
   ctg gcc aat ggc gtg gag ctg aga gat aac cag ctg gtg gtg cca tca     384
   Leu Ala Asn Gly Val Glu Leu Arg Asp Asn Gln Leu Val Val Pro Ser
           115                 120                 125
   gag ggc ctg tac ctc atc tac tcc cag gtc ctc ttc aag ggc caa ggc     432
   Glu Gly Leu Tyr Leu Ile Tyr Ser Gln Val Leu Phe Lys Gly Gln Gly
       130                 135                 140
   tgc ccc tcc acc cat gtg ctc ctc acc cac acc atc agc cgc atc gcc     480
   Cys Pro Ser Thr His Val Leu Leu Thr His Thr Ile Ser Arg Ile Ala
   145                 150                 155                 160
   gtc tcc tac cag acc aag gtc aac ctc ctc tct gcc atc aag agc ccc     528
   Val Ser Tyr Gln Thr Lys Val Asn Leu Leu Ser Ala Ile Lys Ser Pro
                   165                 170                 175
   tgc cag agg gag acc cca gag ggg gct gag gcc aag ccc tgg tat gag     576
   Cys Gln Arg Glu Thr Pro Glu Gly Ala Glu Ala Lys Pro Trp Tyr Glu
               180                 185                 190
   ccc atc tat ctg gga ggg gtc ttc cag ctg gag aag ggt gac cga ctc     624
   Pro Ile Tyr Leu Gly Gly Val Phe Gln Leu Glu Lys Gly Asp Arg Leu
           195                 200                 205
   agc gct gag atc aat cgg ccc gac tat ctc gac ttt gcc gag tct ggg     672
   Ser Ala Glu Ile Asn Arg Pro Asp Tyr Leu Asp Phe Ala Glu Ser Gly
       210                 215                 220
   cag gtc tac ttt ggg atc att gcc ctg tga                             702
   Gln Val Tyr Phe Gly Ile Ile Ala Leu  *
   225                 230
<210> 8
   <211> 233
   <212> PRT
   <213> Homo sapiens
<400> 8
   Met Ser Thr Glu Ser Met Ile Arg Asp Val Glu Leu Ala Glu Glu Ala
    1               5                  10                  15
   Leu Pro Lys Lys Thr Gly Gly Pro Gln Gly Ser Arg Arg Cys Leu Phe
               20                  25                  30
   Leu Ser Leu Phe Ser Phe Leu Ile Val Ala Gly Ala Thr Thr Leu Phe
           35                  40                  45
   Cys Leu Leu His Phe Gly Val Ile Gly Pro Gln Arg Glu Glu Phe Pro
       50                  55                  60
   Arg Asp Leu Ser Leu Ile Ser Pro Leu Ala Gln Ala Val Arg Ser Ser
   65                  70                  75                  80
   Ser Arg Thr Pro Ser Asp Lys Pro Val Ala His Val Val Ala Asn Pro
                   85                  90                  95
   Gln Ala Glu Gly Gln Leu Gln Trp Leu Asn Arg Arg Ala Asn Ala Leu
               100                 105                 110
   Leu Ala Asn Gly Val Glu Leu Arg Asp Asn Gln Leu Val Val Pro Ser
           115                 120                 125
   Glu Gly Leu Tyr Leu Ile Tyr Ser Gln Val Leu Phe Lys Gly Gln Gly
       130                 135                 140
   Cys Pro Ser Thr His Val Leu Leu Thr His Thr Ile Ser Arg Ile Ala
   145                 150                 155                 160
   Val Ser Tyr Gln Thr Lys Val Asn Leu Leu Ser Ala Ile Lys Ser Pro
                   165                 170                 175
   Cys Gln Arg Glu Thr Pro Glu Gly Ala Glu Ala Lys Pro Trp Tyr Glu
               180                 185                 190
   Pro Ile Tyr Leu Gly Gly Val Phe Gln Leu Glu Lys Gly Asp Arg Leu
           195                 200                 205
   Ser Ala Glu Ile Asn Arg Pro Asp Tyr Leu Asp Phe Ala Glu Ser Gly
       210                 215                 220
   Gln Val Tyr Phe Gly Ile Ile Ala Leu
   225                 230
<210> 9
   <211> 1793
   <212> DNA
   <213> Homo sapiens
<220>
   <221> CDS
   <222> (74)...(1201)
   <223> beta-actin expression (ACTB) RNA coding region
<400> 9
   cgcgtccgcc ccgcgagcac agagcctcgc ctttgccgat ccgccgcccg tccacacccg 60
   ccgccagctc acc atg gat gat gat atc gcc gcg ctc gtc gtc gac aac    109
                  Met Asp Asp Asp Ile Ala Ala Leu Val Val Asp Asn
                  1               5                   10
   ggc tcc ggc atg tgc aag gcc ggc ttc gcg ggc gac gat gcc ccc cgg    157
   Gly Ser Gly Met Cys Lys Ala Gly Phe Ala Gly Asp Asp Ala Pro Arg
           15                  20                  25
   gcc gtc ttc ccc tcc atc gtg ggg cgc ccc agg cac cag ggc gtg atg    205
   Ala Val Phe Pro Ser Ile Val Gly Arg Pro Arg His Gln Gly Val Met
       30                  35                  40
   gtg ggc atg ggt cag aag gat tcc tat gtg ggc gac gag gcc cag agc    253
   Val Gly Met Gly Gln Lys Asp Ser Tyr Val Gly Asp Glu Ala Gln Ser
   45                  50                  55                  60
   aag aga ggc atc ctc acc ctg aag tac ccc atc gag cac ggc atc gtc    301
   Lys Arg Gly Ile Leu Thr Leu Lys Tyr Pro Ile Glu His Gly Ile Val
                   65                  70                  75
   acc aac tgg gac gac atg gag aaa atc tgg cac cac acc ttc tac aat    349
   Thr Asn Trp Asp Asp Met Glu Lys Ile Trp His His Thr Phe Tyr Asn
               80                  85                  90
   gag ctg cgt gtg gct ccc gag gag cac ccc gtg ctg ctg acc gag gcc    397
   Glu Leu Arg Val Ala Pro Glu Glu His Pro Val Leu Leu Thr Glu Ala
                   95                      100                     105
   ccc ctg aac ccc aag gcc aac cgc gag aag atg acc cag atc atg ttt     445
   Pro Leu Asn Pro Lys Ala Asn Arg Glu Lys Met Thr Gln Ile Met Phe
       110                     115                     120
   gag acc ttc aac acc cca gcc atg tac gtt gct atc cag gct gtg cta     493
   Glu Thr Phe Asn Thr Pro Ala Met Tyr Val Ala Ile Gln Ala Val Leu
   125                 130                     135                 140
   tcc ctg tac gcc tct ggc cgt acc act ggc atc gtg atg gac tcc ggt     541
   Ser Leu Tyr Ala Ser Gly Arg Thr Thr Gly Ile Val Met Asp Ser Gly
                   145                     150                     155
   gac ggg gtc acc cac act gtg ccc atc tac gag ggg tat gcc ctc ccc     589
   Asp Gly Val Thr His Thr Val Pro Ile Tyr Glu Gly Tyr Ala Leu Pro
               160                     165                     170
   cat gcc atc ctg cgt ctg gac ctg gct ggc cgg gac ctg act gac tac     637
   His Ala Ile Leu Arg Leu Asp Leu Ala Gly Arg Asp Leu Thr Asp Tyr
           175                     180                     185
   ctc atg aag atc ctc acc gag cgc ggc tac agc ttc acc acc acg gcc     685
   Leu Met Lys Ile Leu Thr Glu Arg Gly Tyr Ser Phe Thr Thr Thr Ala
       190                     195                     200
   gag cgg gaa atc gtg cgt gac att aag gag aag ctg tgc tac gtc gcc     733
   Glu Arg Glu Ile Val Arg Asp Ile Lys Glu Lys Leu Cys Tyr Val Ala
   205                 210                     215                 220
   ctg gac ttc gag caa gag atg gcc acg gct gct tcc agc tcc tcc ctg     781
   Leu Asp Phe Glu Gln Glu Met Ala Thr Ala Ala Ser Ser Ser Ser Leu
                   225                     230                     235
   gag aag agc tac gag ctg cct gac ggc cag gtc atc acc att ggc aat     829
   Glu Lys Ser Tyr Glu Leu Pro Asp Gly Gln Val Ile Thr Ile Gly Asn
               240                     245                     250
   gag cgg ttc cgc tgc cct gag gca ctc ttc cag cct tcc ttc ctg ggc     877
   Glu Arg Phe Arg Cys Pro Glu Ala Leu Phe Gln Pro Ser Phe Leu Gly
           255                     260                     265
   atg gag tcc tgt ggc atc cac gaa act acc ttc aac tcc atc atg aag     925
   Met Glu Ser Cys Gly Ile His Glu Thr Thr Phe Asn Ser Ile Met Lys
       270                     275                     280
   tgt gac gtg gac atc cgc aaa gac ctg tac gcc aac aca gtg ctg tct     973
   Cys Asp Val Asp Ile Arg Lys Asp Leu Tyr Ala Asn Thr Val Leu Ser
   285                 290                     295                 300
   ggc ggc acc acc atg tac cct ggc att gcc gac agg atg cag aag gag     1021
   Gly Gly Thr Thr Met Tyr Pro Gly Ile Ala Asp Arg Met Gln Lys Glu
                   305                     310                     315
   atc act gcc ctg gca ccc agc aca atg aag atc aag atc att gct cct     1069
   Ile Thr Ala Leu Ala Pro Ser Thr Met Lys Ile Lys Ile Ile Ala Pro
               320                     325                     330
   cct gag cgc aag tac tcc gtg tgg atc ggc ggc tcc atc ctg gcc tcg     1117
   Pro Glu Arg Lys Tyr Ser Val Trp Ile Gly Gly Ser Ile Leu Ala Ser
           335                     340                     345
   ctg tcc acc ttc cag cag atg tgg atc agc aag cag gag tat gac gag     1165
   Leu Ser Thr Phe Gln Gln Met Trp Ile Ser Lys Gln Glu Tyr Asp Glu
       350                     355                     360
   tcc ggc ccc tcc atc gtc cac cgc aaa tgc ttc tag gcggactatg          1211
   Ser Gly Pro Ser Ile Val His Arg Lys Cys Phe  *
   365 370 375
   acttagttgc gttacaccct ttcttgacaa aacctaactt gcgcagaaaa caagatgaga 1271
   ttggcatggc tttatttgtt ttttttgttt tgttttggtt tttttttttt ttttggcttg 1331
   actcaggatt taaaaactgg aacggtgaag gtgacagcag tcggttggag cgagcatccc 1391
   ccaaagttca caatgtggcc gaggactttg attgcacatt gttgtttttt taatagtcat 1451
   tccaaatatg agatgcattg ttacaggaag tcccttgcca tcctaaaagc caccccactt 1511
   ctctctaagg agaatggccc agtcctctcc caagtccaca caggggaggt gatagcattg 1571
   ctttcgtgta aattatgtaa tgcaaaattt ttttaatctt cgccttaata cttttttatt 1631
   ttgttttatt ttgaatgatg agccttcgtg cccccccttc cccctttttg tcccccaact 1691
   tgagatgtat gaaggctttt ggtctccctg ggagtgggtg gaggcagcca gggcttacct 1751
   gtacactgac ttgagaccag ttgaataaaa gtgcacacct ta                   1793
<210> 10
   <211> 2242
   <212> DNA
   <213> Homo sapiens
<220>
   <221> CDS
   <222> (51)...(991)
   <223> chemokine (C C motif) receptor 2 (CCR2) mRNA
   coding region
<400> 10
   acagagaaag tggattgaac aaggacgcat ttccccagta catccacaac atg ctg      56
                                                          Met Leu
                                                           1
   tcc aca tct cgt tct cgg ttt atc aga aat acc aac gag agc ggt gaa    104
   Ser Thr Ser Arg Ser Arg Phe Ile Arg Asn Thr Asn Glu Ser Gly Glu
           5                   10                  15
   gaa gtc acc acc ttt ttt gat tat gat tac ggt gct ccc tgt cat aaa    152
   Glu Val Thr Thr Phe Phe Asp Tyr Asp Tyr Gly Ala Pro Cys His Lys
       20                  25                  30
   ttt gac gtg aag caa att ggg gcc caa ctc ctg cct ccg ctc tac tcg    200
   Phe Asp Val Lys Gln Ile Gly Ala Gln Leu Leu Pro Pro Leu Tyr Ser
   35                  40                  45                  50
   ctg gtg ttc atc ttt ggt ttt gtg ggc aac atg ctg gtc gtc ctc atc    248
   Leu Val Phe Ile Phe Gly Phe Val Gly Asn Met Leu Val Val Leu Ile
                   55                  60                  65
   tta ata aac tgc aaa aag ctg aag tgc ttg act gac att tac ctg ctc    296
   Leu Ile Asn Cys Lys Lys Leu Lys Cys Leu Thr Asp Ile Tyr Leu Leu
               70                  75                  80
   aac ctg gcc atc tct gat ctg ctt ttt ctt att act ctc cca ttg tgg    344
   Asn Leu Ala Ile Ser Asp Leu Leu Phe Leu Ile Thr Leu Pro Leu Trp
           85                  90                  95
   gct cac tct gct gca aat gag tgg gtc ttt ggg aat gca atg tgc aaa    392
   Ala His Ser Ala Ala Asn Glu Trp Val Phe Gly Asn Ala Met Cys Lys
       100                 105                 110
   tta ttc aca ggg ctg tat cac atc ggt tat ttt ggc gga atc ttc ttc    440
   Leu Phe Thr Gly Leu Tyr His Ile Gly Tyr Phe Gly Gly Ile Phe Phe
   115                 120                 125                 130
   atc atc ctc ctg aca atc gat aga tac ctg gct att gtc cat gct gtg    488
   Ile Ile Leu Leu Thr Ile Asp Arg Tyr Leu Ala Ile Val His Ala Val
                   135                 140                 145
   ttt gct tta aaa gcc agg acg gtc acc ttt ggg gtg gtg aca agt gtg    536
   Phe Ala Leu Lys Ala Arg Thr Val Thr Phe Gly Val Val Thr Ser Val
               150                 155                 160
   atc acc tgg ttg gtg gct gtg ttt gct tct gtc cca gga atc atc ttt     584
   Ile Thr Trp Leu Val Ala Val Phe Ala Ser Val Pro Gly Ile Ile Phe
           165                 170                 175
   act aaa tgc cag aaa gaa gat tct gtt tat gtc tgt ggc cct tat ttt     632
   Thr Lys Cys Gln Lys Glu Asp Ser Val Tyr Val Cys Gly Pro Tyr Phe
       180                 185                 190
   cca cga gga tgg aat aat ttc cac aca ata atg agg aac att ttg ggg     680
   Pro Arg Gly Trp Asn Asn Phe His Thr Ile Met Arg Asn Ile Leu Gly
   195                 200                 205                 210
   ctg gtc ctg ccg ctg ctc atc atg gtc atc tgc tac tcg gga atc ctg     728
   Leu Val Leu Pro Leu Leu Ile Met Val Ile Cys Tyr Ser Gly Ile Leu
                   215                 220                 225
   aaa acc ctg ctt cgg tgt cga aac gag aag aag agg cat agg gca gtg     776
   Lys Thr Leu Leu Arg Cys Arg Asn Glu Lys Lys Arg His Arg Ala Val
               230                 235                 240
   aga gtc atc ttc acc atc atg att gtt tac ttt ctc ttc tgg act ccc     824
   Arg Val Ile Phe Thr Ile Met Ile Val Tyr Phe Leu Phe Trp Thr Pro
           245                 250                 255
   tat aac att gtc att ctc ctg aac acc ttc cag gaa ttc ttc ggc ctg     872
   Tyr Asn Ile Val Ile Leu Leu Asn Thr Phe Gln Glu Phe Phe Gly Leu
       260                 265                 270
   agt aac tgt gaa agc acc agt caa ctg gac caa gcc acg cag gtg aca     920
   Ser Asn Cys Glu Ser Thr Ser Gln Leu Asp Gln Ala Thr Gln Val Thr
   275                 280                 285                 290
   gag act ctt ggg atg act cac tgc tgc atc aat ccc atc atc tat gcc     968
   Glu Thr Leu Gly Met Thr His Cys Cys Ile Asn Pro Ile Ile Tyr Ala
                   295                 300                 305
   ttc gtt ggg gag aag ttc aga ag cctttttcac atagctcttg gctgtaggat   1021
   Phe Val Gly Glu Lys Phe Arg
               310
   tgccccactc caaaaaccag tgtgtggagg tccaggagtg agaccaggaa agaatgtgaa 1081
   agtgactaca caaggactcc tcgatggtcg tggaaaagga aagtcaattg gcagagcccc 1141
   tgaagccagt cttcaggaca aagaaggagc ctagagacag aaatgacaga tctctgcttt 1201
   ggaaatcaca cgtctggctt cacagatgtg tgattcacag tgtgaatctt ggtgtctacg 1261
   ttaccaggca ggaaggctga gaggagagag actccagctg ggttggaaaa cagtattttc 1321
   caaactacct tccagttcct catttttgaa tacaggcata gagttcagac tttttttaaa 1381
   tagtaaaaat aaaattaaag ctgaaaactg caacttgtaa atgtggtaaa gagttagttt 1441
   gagttactat catgtcaaac gtgaaaatgc tgtattagtc acagagataa ttctagcttt 1501
   gagcttaaga attttgagca ggtggtatgt ttgggagact gctgagtcaa cccaatagtt 1561
   gttgattggc aggagttgga agtgtgtgat ctgtgggcac attagcctat gtgcatgcag 1621
   catctaagta atgatgtcgt ttgaatcaca gtatacgctc catcgctgtc atctcagctg 1681
   gatctccatt ctctcaggct tgctgccaaa agccttttgt gttttgtttt gtatcattat 1741
   gaagtcatgc gtttaatcac attcgagtgt ttcagtgctt cgcagatgtc cttgatgctc 1801
   atattgttcc ctattttgcc agtgggaact cctaaatcaa attggcttct aatcaaagct 1861
   tttaaaccct attggtaaag aatggaaggt ggagaagctc cctgaagtaa gcaaagactt 1921
   tcctcttagt cgagccaagt taagaatgtt cttatgttgc ccagtgtgtt tctgatctga 1981
   tgcaagcaag aaacactggg cttctagaac caggcaactt gggaactaga ctcccaagct 2041
   ggactatggc tctactttca ggccacatgg ctaaagaagg tttcagaaag aagtggggac 2101
   agagcagaac tttcaccttc atatatttgt atgatcctaa tgaatgcata aaatgttaag 2161
   ttgatggtga tgaaatgtaa atactgtttt taacaactat gatttggaaa ataaatcaat 2221
   gctataacta tgttgataaa a                                           2242
<210> 11
   <211> 618
   <212> DNA
   <213> Homo sapiens
<220>
   <221> CDS
   <222> (1)...(618)
   <223> Lymphotoxin alpha (LTA) MRNA coding region
<400> 11
   atg aca cca cct gaa cgt ctc ttc ctc cca agg gtg tgt ggc acc acc    48
   Met Thr Pro Pro Glu Arg Leu Phe Leu Pro Arg Val Cys Gly Thr Thr
    1               5                  10                  15
   cta cac ctc ctc ctt ctg ggg ctg ctg ctg gtt ctg ctg cct ggg gcc    96
   Leu His Leu Leu Leu Leu Gly Leu Leu Leu Val Leu Leu Pro Gly Ala
               20                  25                  30
   cag ggg ctc cct ggt gtt ggc ctc aca cct tca gct gcc cag act gcc    144
   Gln Gly Leu Pro Gly Val Gly Leu Thr Pro Ser Ala Ala Gln Thr Ala
           35                  40                  45
   cgt cag cac ccc aag atg cat ctt gcc cac agc acc ctc aaa cct gct    192
   Arg Gln His Pro Lys Met His Leu Ala His Ser Thr Leu Lys Pro Ala
       50                  55                  60
   gct cac ctc att gga gac ccc agc aag cag aac tca ctg ctc tgg aga    240
   Ala His Leu Ile Gly Asp Pro Ser Lys Gln Asn Ser Leu Leu Trp Arg
   65                  70                  75                  80
   gca aac acg gac cgt gcc ttc ctc cag gat ggt ttc tcc ttg agc aac    288
   Ala Asn Thr Asp Arg Ala Phe Leu Gln Asp Gly Phe Ser Leu Ser Asn
                   85                  90                  95
   aat tct ctc ctg gtc ccc acc agt ggc atc tac ttc gtc tac tcc cag    336
   Asn Ser Leu Leu Val Pro Thr Ser Gly Ile Tyr Phe Val Tyr Ser Gln
               100                 105                 110
   gtg gtc ttc tct ggg aaa gcc tac tct ccc aag gcc acc tcc tcc cca    384
   Val Val Phe Ser Gly Lys Ala Tyr Ser Pro Lys Ala Thr Ser Ser Pro
           115                 120                 125
   ctc tac ctg gcc cat gag gtc cag ctc ttc tcc tcc cag tac ccc ttc    432
   Leu Tyr Leu Ala His Glu Val Gln Leu Phe Ser Ser Gln Tyr Pro Phe
       130                 135                 140
   cat gtg cct ctc ctc agc tcc cag aag atg gtg tat cca ggg ctg cag    480
   His Val Pro Leu Leu Ser Ser Gln Lys Met Val Tyr Pro Gly Leu Gln
   145                 150                 155                 160
   gaa ccc tgg ctg cac tcg atg tac cac ggg gct gcg ttc cag ctc acc    528
   Glu Pro Trp Leu His Ser Met Tyr His Gly Ala Ala Phe Gln Leu Thr
                   165                 170                 175
   cag gga gac cag cta tcc acc cac aca gat ggc atc ccc cac cta gtc    576
   Gln Gly Asp Gln Leu Ser Thr His Thr Asp Gly Ile Pro His Leu Val
               180                 185                 190
   ctc agc cct agt act gtc ttc ttt gga gcc ttc gct ctg tag            618
   Leu Ser Pro Ser Thr Val Phe Phe Gly Ala Phe Ala Leu  *
           195                 200                 205
<210> 12
   <211> 894
   <212> DNA
   <213> Homo sapiens
<220>
   <221> CDS
   <222> (9)...(743)
   <223> Lymphotoxin beta (LTB) mRNA coding region
<400> 12
   cagtctca atg ggg gca ctg ggg ctg gag ggc agg ggt ggg agg ctc cag    50
            Met Gly Ala Leu Gly Leu Glu Gly Arg Gly Gly Arg Leu Gln
             1               5                   10
   ggg agg ggt tcc ctc ctg cta gct gtg gca gga gcc act tct ctg gtg    98
   Gly Arg Gly Ser Leu Leu Leu Ala Val Ala Gly Ala Thr Ser Leu Val
    15                  20                  25                  30
   acc ttg ttg ctg gcg gtg cct atc act gtc ctg gct gtg ctg gcc tta    146
   Thr Leu Leu Leu Ala Val Pro Ile Thr Val Leu Ala Val Leu Ala Leu
                    35                  40                  45
   gtg ccc cag gat cag gga gga ctg gta acg gag acg gcc gac ccc ggg    194
   Val Pro Gln Asp Gln Gly Gly Leu Val Thr Glu Thr Ala Asp Pro Gly
                50                  55                  60
   gca cag gcc cag caa gga ctg ggg ttt cag aag ctg cca gag gag gag    242
   Ala Gln Ala Gln Gln Gly Leu Gly Phe Gln Lys Leu Pro Glu Glu Glu
            65                  70                  75
   cca gaa aca gat ctc agc ccc ggg ctc cca gct gcc cac ctc ata ggc    290
   Pro Glu Thr Asp Leu Ser Pro Gly Leu Pro Ala Ala His Leu Ile Gly
        80                  85                  90
   gct ccg ctg aag ggg cag ggg cta ggc tgg gag acg acg aag gaa cag    338
   Ala Pro Leu Lys Gly Gln Gly Leu Gly Trp Glu Thr Thr Lys Glu Gln
    95                 100                 105                 110
   gcg ttt ctg acg agc ggg acg cag ttc tcg gac gcc gag ggg ctg gcg    386
   Ala Phe Leu Thr Ser Gly Thr Gln Phe Ser Asp Ala Glu Gly Leu Ala
                   115                 120                 125
   ctc ccg cag gac ggc ctc tat tac ctc tac tgt ctc gtc ggc tac cgg    434
   Leu Pro Gln Asp Gly Leu Tyr Tyr Leu Tyr Cys Leu Val Gly Tyr Arg
               130                 135                 140
   ggc cgg gcg ccc cct ggc ggc ggg gac ccc cag ggc cgc tcg gtc acg    482
   Gly Arg Ala Pro Pro Gly Gly Gly Asp Pro Gln Gly Arg Ser Val Thr
           145                 150                 155
   ctg cgc agc tct ctg tac cgg gcg ggg ggc gcc tac ggg ccg ggc act    530
   Leu Arg Ser Ser Leu Tyr Arg Ala Gly Gly Ala Tyr Gly Pro Gly Thr
       160                 165                 170
   ccc gag ctg ctg ctc gag ggc gcc gag acg gtg act cca gtg ctg gac    578
   Pro Glu Leu Leu Leu Glu Gly Ala Glu Thr Val Thr Pro Val Leu Asp
   175                 180                 185                 190
   ccg gcc agg aga caa ggg tac ggg cct ctc tgg tac acg agc gtg ggg    626
   Pro Ala Arg Arg Gln Gly Tyr Gly Pro Leu Trp Tyr Thr Ser Val Gly
                   195                 200                 205
   ttc ggc ggc ctg gtg cag ctc cgg agg ggc gag agg gtg tac gtc aac    674
   Phe Gly Gly Leu Val Gln Leu Arg Arg Gly Glu Arg Val Tyr Val Asn
               210                 215                 220
   atc agt cac ccc gat atg gtg gac ttc gcg aga ggg aag acc ttc ttt    722
   Ile Ser His Pro Asp Met Val Asp Phe Ala Arg Gly Lys Thr Phe Phe
           225                 230                 235
   ggg gcc gtg atg gtg ggg tga gggaatatga gtgcgtggtg cgagtgcgtg       773
   Gly Ala Val Met Val Gly  *
       240
   aatattgggg gcccggacgc ccaggacccc atggcagtgg gaaaaatgta ggagactgtt 833
   tggaaattga ttttgaacct gatgaaaata aagaatggaa agcttcagtg ctgccgataa 893
   a                                                                 894
<210> 13
   <211> 327
   <212> DNA
   <213> Homo sapiens
<220>
   <221> CDS
   <222> (1)...(327)
   <223> FKBP1A open reading frame for FK506 Binding
   Protein 1A (macrophilin 12) mRNA coding region
<400> 13
   atg gga gtg cag gtg gaa acc atc tcc cca gga gac ggg cgc acc ttc     48
   Met Gly Val Gln Val Glu Thr Ile Ser Pro Gly Asp Gly Arg Thr Phe
    1               5                  10                  15
   ccc aag cgc ggc cag acc tgc gtg gtg cac tac acc ggg atg ctt gaa     96
   Pro Lys Arg Gly Gln Thr Cys Val Val His Tyr Thr Gly Met Leu Glu
               20                  25                  30
   gat gga aag aaa ttt gat tcc tcc cgg gac aga aac aag ccc ttt aag    144
   Asp Gly Lys Lys Phe Asp Ser Ser Arg Asp Arg Asn Lys Pro Phe Lys
           35                  40                  45
   ttt atg cta ggc aag cag gag gtg atc cga ggc tgg gaa gaa ggg gtt    192
   Phe Met Leu Gly Lys Gln Glu Val Ile Arg Gly Trp Glu Glu Gly Val
       50                  55                  60
   gcc cag atg agt gtg ggt cag aga gcc aaa ctg act ata tct cca gat    240
   Ala Gln Met Ser Val Gly Gln Arg Ala Lys Leu Thr Ile Ser Pro Asp
   65                  70                  75                  80
   tat gcc tat ggt gcc act ggg cac cca ggc atc atc cca cca cat gcc    288
   Tyr Ala Tyr Gly Ala Thr Gly His Pro Gly Ile Ile Pro Pro His Ala
                   85                  90                  95
   act ctc gtc ttc gat gtg gag ctt cta aaa ctg gaa tga                327
   Thr Leu Val Phe Asp Val Glu Leu Leu Lys Leu Glu  *
               100                 105
<210> 14
   <211> 21
   <212> DNA
   <213> Homo sapiens
<220>
   <221> primer_bind
   <222> (1)...(21)
   <223> Amplification primer for human LTA - forward
<400> 14
   acaccacctg aacgtctctt c 21
<210> 15
   <211> 21
   <212> DNA
   <213> Homo sapiens
<220>
   <221> primer_bind
   <222> (1)...(21)
   <223> Amplification primer for human LTA - reverse
<400> 15
   tctcaatccc tgaggaagtg g 21
<210> 16
   <211> 24
   <212> DNA
   <213> Homo sapiens
<220>
   <221> primer_bind
   <222> (1)...(24)
   <223> sequencing primer for human LTA - forward
<400> 16
   tcagccaaac cttgagccct agag 24
<210> 17
   <211> 34
   <212> DNA
   <213> Homo sapiens
<220>
   <221> primer_bind
   <222> (1)...(34)
   <223> sequencing primer for human LTA - reverse
<400> 17
   atgtttacca atgaggtgag cagcaggttt gcgg 34
<210> 18
   <211> 20
   <212> DNA
   <213> Homo sapiens
<220>
   <221> primer_bind
   <222> (1)...(20)
   <223> Amplification primer for human TNF - forward
<400> 18
   tgggagtgag aacttcccag 20
<210> 19
   <211> 20
   <212> DNA
   <213> Homo sapiens
<220>
   <221> primer_bind
   <222> (1)...(20)
   <223> Amplification primer for human TNF - reverse
<400> 19
   tgagctcatc tggaggaagc 20
<210> 20
   <211> 20
   <212> DNA
   <213> Homo sapiens
<220>
   <221> primer_bind
   <222> (1)...(20)
   <223> Sequencing primer for human TNF - forward
<400> 20
   tgggagtgag aacttcccag 20
<210> 21
   <211> 20
   <212> DNA
   <213> Homo sapiens
<220>
   <221> primer_bind
   <222> (1)...(20)
   <223> sequencing primer for human TNF - reverse
<400> 21
   cttaaacgtc ccctgtattc 20
<210> 22
   <211> 894
   <212> DNA
   <213> Homo sapiens
<220>
   <221> variation
   <222> (9)...(743)
   <223> LTB*1 (G) polymorphism coding region
<400> 22
   cagtctcaat gggggcactg gggctggagg gcaggggtgg gaggctccag gggaggggtt 60
   ccctcctgct agctgtggca ggagccactt ctctggtgac cttgttgctg gcggtgccta 120
   tcactgtcct ggctgtgctg gccttagtgc cccaggatca gggaggactg gtaacggaga 180
   cggccgaccc cggggcacag gcccagcaag gactggggtt tcagaagctg ccagaggagg 240
   agccagaaac agatctcagc cccgggctcc cagctgccca cctcataggc gctccgctga 300
   aggggcaggg gctaggctgg gagacgacga aggaacaggc gtttctgacg agcgggacgc 360
   agttctcgga cgccgagggg ctggcgctcc cgcaggacgg cctctattac ctctactgtc 420
   tcgtcggcta ccggggccgg gcgccccctg gcggcgggga cccccagggc cgctcggtca 480
   cgctgcgcag ctctctgtac cgggcggggg gcgcctacgg gccgggcact cccgagctgc 540
   tgctcgaggg cgccgagacg gtgactccag tgctggaccc ggccaggaga caagggtacg 600
   ggcctctctg gtacacgagc gtggggttcg gcggcctggt gcagctccgg aggggcgaga 660
   gggtgtacgt caacatcagt caccccgata tggtggactt cgcgagaggg aagaccttct 720
   ttggggccgt gatggtgggg tgagggaata tgagtgcgtg gtgcgagtgc gtgaatattg 780
   ggggcccgga cgcccaggac cccatggcag tgggaaaaat gtaggagact gtttggaaat 840
   tgattttgaa cctgatgaaa ataaagaatg gaaagcttca gtgctgccga taaa       894